(19) Europäisches Patentamt
European Patent Office
Office européen des brevets

(11) EP 2 726 507 B1

(12) **EUROPÄISCHE PATENTSCHRIFT**

(45) Veröffentlichungstag und Bekanntmachung des Hinweises auf die Patenterteilung:
**21.11.2018 Patentblatt 2018/47**

(21) Anmeldenummer: **12729996.4**

(22) Anmeldetag: **29.06.2012**

(51) Int Cl.:
***C07K 16/28*** (2006.01) ***C07K 16/30*** (2006.01)

(86) Internationale Anmeldenummer:
**PCT/EP2012/062716**

(87) Internationale Veröffentlichungsnummer:
**WO 2013/001065 (03.01.2013 Gazette 2013/01)**

(54) **ANTIKÖRPER GEGEN DAS PROSTATA-SPEZIFISCHE STAMMZELLANTIGEN UND DESSEN VERWENDUNG**

ANTIBODIES AGAINST PROSTATE-SPECIFIC STEM CELL ANTIGEN AND USE THEREOF

ANTICORPS CONTRE L'ANTIGÈNE DES CELLULES SOUCHES PROSTATIQUES ET UTILISATION DUDIT ANTICORPS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.06.2011 DE 102011118022**

(43) Veröffentlichungstag der Anmeldung:
**07.05.2014 Patentblatt 2014/19**

(73) Patentinhaber: **GEMoaB Monoclonals GmbH
01307 Dresden (DE)**

(72) Erfinder: **BACHMANN, Michael
65779 Kelkheim (DE)**

(74) Vertreter: **Kailuweit & Uhlemann Patentanwälte Partnerschaft mbB
Bamberger Straße 49
01187 Dresden (DE)**

(56) Entgegenhaltungen:
**WO-A1-01/05427 WO-A2-01/40309
WO-A2-2009/032949**

- **ERIC J LEPIN ET AL: "An affinity matured minibody for PET imaging of prostate stem cell antigen (PSCA)-expressing tumors", EUROPEAN JOURNAL OF NUCLEAR MEDICINE AND MOLECULAR IMAGING, SPRINGER, BERLIN, DE, Bd. 37, Nr. 8, 1. April 2010 (2010-04-01), Seiten 1529-1538, XP019841981, ISSN: 1619-7089**
- **Agnieszka Morgenroth: "Herstellung chimärer Rrezeptoren zur tumorspezifischen Armierung polyklonaler, zytotoxischer T-Lymphozyten", DISSERTATION ZUR ERLANGUNG DES AKADEMISCHEN GRADES DOCTOR RERUM NATURALIUM, TU DRESDEN, 7. Dezember 2005 (2005-12-07), XP55037578, Gefunden im Internet: URL:http://www.qucosa.de/fileadmin/data/qucosa/documents/1412/1134590060614-4888.pdf [gefunden am 2012-09-07]**
- **A. FELDMANN ET AL: "Novel Humanized and Highly Efficient Bispecific Antibodies Mediate Killing of Prostate Stem Cell Antigen-Expressing Tumor Cells by CD8+ and CD4+ T Cells", THE JOURNAL OF IMMUNOLOGY, Bd. 189, Nr. 6, 15. September 2012 (2012-09-15), Seiten 3249-3259, XP55037485, ISSN: 0022-1767, DOI: 10.4049/jimmunol.1200341**
- **ANJA FELDMANN ET AL: "Retargeting of T cells to prostate stem cell antigen expressing tumor cells: Comparison of different antibody formats", THE PROSTATE, Bd. 71, Nr. 9, 15. Juni 2011 (2011-06-15), Seiten 998-1011, XP55008882, ISSN: 0270-4137, DOI: 10.1002/pros.21315**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- SCHWARZER A ED - SCHWARZER ET AL: "Herstellung und Charakterisierung rekombinanter Antikörper für eine adjuvante Immuntherapie PSCA-positiver Tumore", DISSERTATIONSSCHRIFT ZUR ERLANGUNG EINES DOCTOR MEDICINAE (DR.MED.) DER MEDIZINISCHEN FAKULTÄT CARL GUSTAV CARUS DER TECHNISCHEN UNIVERSITÄT DRESDEN,, [Online] 1. Januar 2006 (2006-01-01), Seiten 22-24,96, XP002662580, Gefunden im Internet: URL:http://swbplus.bsz-bw.de/bsz276658973inh.pdf>
- SIOBHAN O'BRIEN ET AL: "Humanising Antibodies by CDR Grafting", ANTIBODY ENGINEERING, XX, XX, 1 January 2001 (2001-01-01), page 582, XP002420989,

SEQUENCE LISTING

<110> Technische Universität Dresden

<120> Antikörper gegen das Prostata-spezifische Stammzellantigen und dessen Verwendung

<130> 00017P0179DEWO

<150> DE 10 2011 118 022.6
<151> 2011-06-30

<160> 172

<170> PatentIn version 3.5

<210> 1
<211> 11
<212> PRT
<213> Mus musculus

<400> 1

```
Gly Thr Ser Gln Asp Ile Asn Asn Tyr Leu Asn
1               5                   10
```

<210> 2
<211> 7
<212> PRT
<213> Mus musculus

<400> 2

```
Tyr Thr Ser Arg Leu His Ser
1               5
```

<210> 3
<211> 9
<212> PRT
<213> Mus musculus

<400> 3

```
Gln Gln Ser Lys Thr Leu Pro Trp Thr
1               5
```

<210> 4
<211> 5
<212> PRT
<213> Mus musculus

<400> 4

```
Ser Tyr Ser Met Ser
1               5
```

<210> 5
<211> 17
<212> PRT
<213> Mus musculus

<400> 5

Tyr Ile Asn Asp Ser Gly Gly Ser Thr Phe Tyr Pro Asp Thr Val Lys
1 5 10 15

Gly

<210> 6
<211> 13
<212> PRT
<213> Mus musculus

<400> 6

Arg Met Tyr Tyr Gly Asn Ser His Trp His Phe Asp Val
1 5 10

<210> 7
<211> 33
<212> DNA
<213> Mus musculus

<400> 7
gggacaagtc aggacattaa caattattta aac 33

<210> 8
<211> 33
<212> DNA
<213> Homo sapiens

<400> 8
ggtacatccc aagacatcaa taattatctc aac 33

<210> 9
<211> 21
<212> DNA
<213> Mus musculus

<400> 9
tacacatcaa gattacactc g 21

<210> 10
<211> 21
<212> DNA
<213> Homo sapiens

<400> 10
tacacatcca ggctgcattc c 21

<210> 11
<211> 27
<212> DNA
<213> Mus musculus

<400> 11
caacagtcta agacgcttcc gtggacg 27

<210> 12
<211> 27
<212> DNA

<213> Homo sapiens

<400> 12
cagcagtcaa agacattacc atggaca 27

<210> 13
<211> 15
<212> DNA
<213> Mus musculus

<400> 13
tcctattcca tgtct 15

<210> 14
<211> 15
<212> DNA
<213> Homo sapiens

<400> 14
agttactcta tgtca 15

<210> 15
<211> 51
<212> DNA
<213> Mus musculus

<400> 15
tacattaatg atagtggtgg tagcaccttt tatccagaca ctgtgaaggg c 51

<210> 16
<211> 51
<212> DNA
<213> Homo sapiens

<400> 16
tacattaatg attcaggtgg aagtacattc tatccggaca cggttaaagg t 51

<210> 17
<211> 39
<212> DNA
<213> Mus musculus

<400> 17
cggatgtact acggtaatag ccactggcac ttcgatgtc 39

<210> 18
<211> 39
<212> DNA
<213> Homo sapiens

<400> 18
cgtatgtatt atggcaatag tcactggcac tttgacgtc 39

<210> 19
<211> 348
<212> DNA
<213> Mus musculus

<400> 19

```
gatattgtga tgacacagtc tacatcctcc ctgtctgcct ctctgggaga cagagtcacc      60
atcaattgcg ggacaagtca ggacattaac aattatttaa actggtatca gcagaaacca     120
gatggaagtg ttaaactcct gatctactac acatcaagat tacactcggg agtcccatcc     180
aggttcagtg gcagtgggtc tggaacagat tattctctca ccattagcaa cctggagcaa     240
ggggatattg ccacttactt ctgccaacag tctaagacgc ttccgtggac gttcggtgga     300
ggcaccaagc tggaactcaa acgggctgat gctgcaccaa ctgtatcc                   348
```

<210> 20
<211> 116
<212> PRT
<213> Mus musculus

<400> 20

```
Asp Ile Val Met Thr Gln Ser Thr Ser Ser Leu Ser Ala Ser Leu Gly
1               5                   10                  15

Asp Arg Val Thr Ile Asn Cys Gly Thr Ser Gln Asp Ile Asn Asn Tyr
            20                  25                  30

Leu Asn Trp Tyr Gln Gln Lys Pro Asp Gly Ser Val Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Glu Gln
65                  70                  75                  80

Gly Asp Ile Ala Thr Tyr Phe Cys Gln Gln Ser Lys Thr Leu Pro Trp
                85                  90                  95

Thr Phe Gly Gly Gly Thr Lys Leu Glu Leu Lys Arg Ala Asp Ala Ala
            100                 105                 110

Pro Thr Val Ser
        115
```

<210> 21
<211> 363
<212> DNA
<213> Mus musculus

<400> 21

```
gtacagctgc aggagtcagg gggagtttta gtgcagcctg gagggtccct gaaactctcc        60

tgtgcagcct ctggattcac tttcagttcc tattccatgt cttggattcg ccagactcca       120

gacaggaggc tggagtgggt cgcatacatt aatgatagtg gtggtagcac cttttatcca       180

gacactgtga agggccgatt ctccatctcc agagacaatg ccaagaacac cctgtacctc       240

caaataagca gactgaggtc tggggacacg gccatttatt actgttcaag acggatgtac       300

tacggtaata gccactggca cttcgatgtc tggggcgcag ggaccacggt caccgtctcc       360

tca                                                                     363
```

<210> 22
<211> 121
<212> PRT
<213> Mus musculus

<400> 22

```
Val Gln Leu Gln Glu Ser Gly Gly Val Leu Val Gln Pro Gly Gly Ser
1               5                   10                  15

Leu Lys Leu Ser Cys Ala Ala Ser Gly Phe Thr Phe Ser Ser Tyr Ser
            20                  25                  30

Met Ser Trp Ile Arg Gln Thr Pro Asp Arg Arg Leu Glu Trp Val Ala
        35                  40                  45

Tyr Ile Asn Asp Ser Gly Gly Ser Thr Phe Tyr Pro Asp Thr Val Lys
    50                  55                  60

Gly Arg Phe Ser Ile Ser Arg Asp Asn Ala Lys Asn Thr Leu Tyr Leu
65                  70                  75                  80

Gln Ile Ser Arg Leu Arg Ser Gly Asp Thr Ala Ile Tyr Tyr Cys Ser
                85                  90                  95

Arg Arg Met Tyr Tyr Gly Asn Ser His Trp His Phe Asp Val Trp Gly
            100                 105                 110

Ala Gly Thr Thr Val Thr Val Ser Ser
        115                 120
```

<210> 23
<211> 321
<212> DNA
<213> Homo sapiens

<400> 23

gatatccaga tgactcaaag tcctagttcc ctgtctgcat cagtgggaga ccgggtgacc 60

attacatgcg gtacatccca agacatcaat aattatctca actggtatca gcagaagcca 120

ggcaaagttc ctaagttatt aatctactac acatccaggc tgcattccgg ggtgccctcc 180

cgcttttcgg gctccgggtc gggaaccgac tttaccctaa ccatatcttc cctgcagcct 240

gaagacgttg caacgtacta ttgtcagcag tcaaagacat taccatggac atttggtggt 300

gggacgcaac tcactgtact t 321

<210> 24
<211> 107
<212> PRT
<213> Homo sapiens

<400> 24

Asp Ile Gln Met Thr Gln Ser Pro Ser Ser Leu Ser Ala Ser Val Gly
1 5 10 15

Asp Arg Val Thr Ile Thr Cys Gly Thr Ser Gln Asp Ile Asn Asn Tyr
20 25 30

Leu Asn Trp Tyr Gln Gln Lys Pro Gly Lys Val Pro Lys Leu Leu Ile
35 40 45

Tyr Tyr Thr Ser Arg Leu His Ser Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Val Ala Thr Tyr Tyr Cys Gln Gln Ser Lys Thr Leu Pro Trp
85 90 95

Thr Phe Gly Gly Gly Thr Gln Leu Thr Val Leu
100 105

<210> 25
<211> 366
<212> DNA
<213> Homo sapiens

<400> 25

```
caggtgcagc tagtggagtc cggtggcggc ctcgttaagc cgggcggatc gctgcgcctt        60
tcatgtgccg catcaggatt cacattctcc agttactcta tgtcatggat tcggcaggca       120
cctggcaagg gattggaatg ggtctcgtac attaatgatt caggtggaag tacattctat       180
ccggacacgg ttaaaggtag atttaccatc agccgtgata acgcgaagaa tagcttgtac       240
ttacagatga atagcctgcg tgcagaggat actgctgtat attattgcgc tcgacgtatg       300
tattatggca atagtcactg gcactttgac gtctggggcc agggcacgac agttactgtc       360
tcttcg                                                                  366
```

<210> 26
<211> 122
<212> PRT
<213> Homo sapiens

<400> 26

```
Gln Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Ser Met Ser Trp Ile Arg Gln Ala Pro Gly Lys Gly Leu Glu Trp Val
        35                  40                  45

Ser Tyr Ile Asn Asp Ser Gly Gly Ser Thr Phe Tyr Pro Asp Thr Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Arg Met Tyr Tyr Gly Asn Ser His Trp His Phe Asp Val Trp
            100                 105                 110

Gly Gln Gly Thr Thr Val Thr Val Ser Ser
        115                 120
```

<210> 27
<211> 11
<212> PRT
<213> Mus musculus

<400> 27

Arg Thr Ser Gln Asp Ile Ser Asn Tyr Leu Asn
1               5                   10

<210> 28
<211> 7
<212> PRT
<213> Mus musculus

<400> 28

Tyr Thr Leu Lys Leu Asn Ser
1               5

<210> 29
<211> 9
<212> PRT
<213> Mus musculus

<400> 29

Gln Gln Ser Lys Thr Leu Pro Trp Thr
1               5

<210> 30
<211> 5
<212> PRT
<213> Mus musculus

<400> 30

Ser Tyr Thr Met Ser
1               5

<210> 31
<211> 17
<212> PRT
<213> Mus musculus

<400> 31

Tyr Ile His Asn Gly Gly Gly His Thr Tyr Tyr Pro Asp Thr Ile Lys
1               5                   10                  15

Gly

<210> 32
<211> 13
<212> PRT
<213> Mus musculus

<400> 32

Arg Met Tyr Tyr Gly Asn Ser His Trp Tyr Phe Asp Val
1               5                   10

<210> 33
<211> 33

<212> DNA
<213> Mus musculus

<400> 33
aggacaagcc aggacattag caactattta aac 33

<210> 34
<211> 33
<212> DNA
<213> Homo sapiens

<400> 34
cggacctcac aagacatcag caactatctg aat 33

<210> 35
<211> 21
<212> DNA
<213> Mus musculus

<400> 35
tacacattaa aattaaattc a 21

<210> 36
<211> 21
<212> DNA
<213> Homo sapiens

<400> 36
tacacattaa aactaaattc c 21

<210> 37
<211> 27
<212> DNA
<213> Mus musculus

<400> 37
caacagagta aaacacttcc gtggacg 27

<210> 38
<211> 27
<212> DNA
<213> Homo sapiens

<400> 38
cagcagtcca aaaccctgcc ttggacc 27

<210> 39
<211> 15
<212> DNA
<213> Mus musculus

<400> 39
tcctatacca tgtct 15

<210> 40
<211> 15
<212> DNA
<213> Homo sapiens

<400> 40
agctatacaa tgtct 15

<210> 41
<211> 51
<212> DNA
<213> Mus musculus

<400> 41
tacattcata atggtggtgg tcacacctac tatccagaca ccataaaggg c 51

<210> 42
<211> 51
<212> DNA
<213> Homo sapiens

<400> 42
tacatccata atggaggcgg tcacacctac tatcctgaca ctatcaaagg a 51

<210> 43
<211> 39
<212> DNA
<213> Mus musculus

<400> 43
cgaatgtact acggtaatag ccactggtac ttcgatgtc 39

<210> 44
<211> 39
<212> DNA
<213> Homo sapiens

<400> 44
cgtatgtact acgggaacag ccactggtac ttcgacgtg 39

<210> 45
<211> 348
<212> DNA
<213> Mus musculus

<400> 45

```
gatattgtga tgacacagtc tccatcctcc ctgtctgcct ctctgggcga cagagtcacc    60
atcaattgca ggacaagcca ggacattagc aactatttaa actggtatca gctgacacca   120
gatggaactg ttaaactcct gatctactac acattaaaat taaattcagg agtcccatca   180
aggttcagtg gcagtgggtc tgggacagat tattctctca ccattaacaa cctggagaaa   240
gaggattttg ccacttattt ttgccaacag agtaaaacac ttccgtggac gttcggtgga   300
ggcaccaagc tggaaatcaa gcgggctgat gctgcaccaa ctgtatcc                348
```

<210> 46
<211> 116
<212> PRT
<213> Mus musculus

<400> 46

```
Asp Ile Val Met Thr Gln Ser Pro Ser Ser Leu Ser Ala Ser Leu Gly
1               5                   10                  15

Asp Arg Val Thr Ile Asn Cys Arg Thr Ser Gln Asp Ile Ser Asn Tyr
            20                  25                  30

Leu Asn Trp Tyr Gln Leu Thr Pro Asp Gly Thr Val Lys Leu Leu Ile
        35                  40                  45

Tyr Tyr Thr Leu Lys Leu Asn Ser Gly Val Pro Ser Arg Phe Ser Gly
    50                  55                  60

Ser Gly Ser Gly Thr Asp Tyr Ser Leu Thr Ile Asn Asn Leu Glu Lys
65                  70                  75                  80

Glu Asp Phe Ala Thr Tyr Phe Cys Gln Gln Ser Lys Thr Leu Pro Trp
                85                  90                  95

Thr Phe Gly Gly Gly Thr Lys Leu Glu Ile Lys Arg Ala Asp Ala Ala
            100                 105                 110

Pro Thr Val Ser
        115
```

<210> 47
<211> 363
<212> DNA
<213> Mus musculus

<400> 47

```
caggtgaagc tgcaggagtc tgggggaggt ttagtgcagc ctggagggtc cctgaaactc      60

tcctgtgtag cctctggatt cactttcagt tcctatacca tgtcttgggt tcgccggact     120

ccagagaaga ggctggaatg ggtcgcatac attcataatg gtggtggtca cacctactat     180

ccagacacca taaagggccg attcaccatc tccagagaca atgccaagaa caccctgttc     240

ctggaaatga gcagtctgaa gtctgaagac acggccatgt attactgtac aagacgaatg     300

tactacggta atagccactg gtacttcgat gtctggggcg cagggacctc ggtcaccgtc     360

tcc                                                                   363
```

<210> 48
<211> 121
<212> PRT
<213> Mus musculus

<400> 48

```
Gln Val Lys Leu Gln Glu Ser Gly Gly Gly Leu Val Gln Pro Gly Gly
1               5                   10                  15

Ser Leu Lys Leu Ser Cys Val Ala Ser Gly Phe Thr Phe Ser Ser Tyr
            20                  25                  30

Thr Met Ser Trp Val Arg Arg Thr Pro Glu Lys Arg Leu Glu Trp Val
        35                  40                  45

Ala Tyr Ile His Asn Gly Gly Gly His Thr Tyr Tyr Pro Asp Thr Ile
    50                  55                  60

Lys Gly Arg Phe Thr Ile Ser Arg Asp Asn Ala Lys Asn Thr Leu Phe
65                  70                  75                  80

Leu Glu Met Ser Ser Leu Lys Ser Glu Asp Thr Ala Met Tyr Tyr Cys
                85                  90                  95

Thr Arg Arg Met Tyr Tyr Gly Asn Ser His Trp Tyr Phe Asp Val Trp

                100                 105                 110

Gly Ala Gly Thr Ser Val Thr Val Ser
        115                 120
```

<210> 49
<211> 321
<212> DNA
<213> Homo sapiens

<400> 49

```
gatatccaga tgacgcaatc accgagttct ctatccgcgt cggttggcga cagagtgact      60
atcacatgtc ggacctcaca agacatcagc aactatctga attggtatca acaaaagcca     120
ggcaaggctc ccaagctatt aatttattac acattaaaac taaattccgg tgtcccatcc     180
agattctcgg gtagcgggtc ggggacggat tttaccttca cgatatcctc cctccagcct     240
gaggacatcg ccacgtacta ctgccagcag tccaaaaccc tgccttggac cttcggtgga     300
gggaccaaag tcgaaattaa g                                               321
```

<210> 50
<211> 107
<212> PRT
<213> Homo sapiens

<400> 50

Asp Ile Gln Met Thr Gln Ser Pro Ser Ser Leu Ser Ala Ser Val Gly
1               5                   10                  15

Asp Arg Val Thr Ile Thr Cys Arg Thr Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Tyr Thr Leu Lys Leu Asn Ser Gly Val Pro Ser Arg Phe Ser Gly
    50                  55                  60

Ser Gly Ser Gly Thr Asp Phe Thr Phe Thr Ile Ser Ser Leu Gln Pro
65                  70                  75                  80

Glu Asp Ile Ala Thr Tyr Tyr Cys Gln Gln Ser Lys Thr Leu Pro Trp
                85                  90                  95

Thr Phe Gly Gly Gly Thr Lys Val Glu Ile Lys
            100                 105

<210> 51
<211> 366
<212> DNA
<213> Homo sapiens

<400> 51

gaggtacagc ttgttgaatc tggaggtggc ctggttcagc ccggcggctc actaaggctt 60

agttgtgccg cttcagggtt cacattttcc agctatacaa tgtcttgggt ccgacaggca 120

ccgggaaaag gactggagtg ggtgagctac atccataatg gaggcggtca cacctactat 180

cctgacacta tcaaaggaag gttcactatc agtcgagata atgcgaagaa ctcactctac 240

ctacagatga acagcctgcg cgccgaagac accgctgtat actattgcgc acgccgtatg 300

tactacggga acagccactg gtacttcgac gtgtggggtc aaggtacgac cgttaccgtg 360

tcttcc 366

<210> 52
<211> 122
<212> PRT
<213> Homo sapiens

<400> 52

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Thr Met Ser Trp Val Arg Gln Ala Pro Gly Lys Gly Leu Glu Trp Val
        35                  40                  45

Ser Tyr Ile His Asn Gly Gly Gly His Thr Tyr Tyr Pro Asp Thr Ile
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Arg Met Tyr Tyr Gly Asn Ser His Trp Tyr Phe Asp Val Trp
            100                 105                 110

Gly Gln Gly Thr Thr Val Thr Val Ser Ser
        115                 120

<210> 53
<211> 318
<212> DNA
<213> Homo sapiens

<400> 53

gaaattgtgt tgacacagtc tccagccacc ctgtctttgt ctccagggga aagagccacc 60

ctctcctgca gtgccagttc aagtgtaagt tacatgcact ggtaccaaca gaaacctggc 120

caggctccca ggctcctcat ctatgacaca tccaaactgg cttctggcat cccagccagg 180

ttcagtggca gtgggtctgg gacagacttc actctcacca tcagcagcct agagcctgaa 240

gattttgcag tttattactg tcagcagtgg agtagtaacc cgctcacgtt cggcggaggg 300

accaaggtgg agatcaaa 318

<210> 54
<211> 30
<212> DNA
<213> Homo sapiens

<400> 54
agtgccagtt caagtgtaag ttacatgcac 30

<210> 55
<211> 21
<212> DNA
<213> Homo sapiens

<400> 55
gacacatcca aactggcttc t 21

<210> 56
<211> 27
<212> DNA
<213> Homo sapiens

<400> 56
cagcagtgga gtagtaaccc gctcacg 27

<210> 57
<211> 106
<212> PRT
<213> Homo sapiens

<400> 57

```
Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Val Ser Tyr Met
            20                  25                  30

His Trp Tyr Gln Gln Lys Pro Gly Gln Ala Pro Arg Leu Leu Ile Tyr
        35                  40                  45

Asp Thr Ser Lys Leu Ala Ser Gly Ile Pro Ala Arg Phe Ser Gly Ser
    50                  55                  60

Gly Ser Gly Thr Asp Phe Thr Leu Thr Ile Ser Ser Leu Glu Pro Glu
65                  70                  75                  80

Asp Phe Ala Val Tyr Tyr Cys Gln Gln Trp Ser Ser Asn Pro Leu Thr

                  85                  90                  95

    Phe Gly Gly Gly Thr Lys Val Glu Ile Lys
                100                 105
```

<210> 58
<211> 10
<212> PRT
<213> Homo sapiens

<400> 58

Ser Ala Ser Ser Ser Val Ser Tyr Met His
1 5 10

<210> 59
<211> 7
<212> PRT
<213> Homo sapiens

<400> 59

Asp Thr Ser Lys Leu Ala Ser
1 5

<210> 60
<211> 9
<212> PRT
<213> Homo sapiens

<400> 60

Gln Gln Trp Ser Ser Asn Pro Leu Thr
1 5

<210> 61
<211> 351
<212> DNA
<213> Homo sapiens

<400> 61

caggttcagc tggtgcagtc tggagctgag gtgaagaagc ctggggcctc agtgaaggtc 60

tcctgtaagg cttctggtta cacctttacc aaatatgtta tacattgggt gcgacaggcc 120

cctggacaag ggcttgagtg gatgggatat attaatcctt acaatgatgt tagtaagtac 180

aatgagaagt tcagaggcag agtcaccatg accacagaca catccacgag cacagcctac 240

atggagctga ggagcctgag atctgacgac acggccgtgt attactgtgc gagaaataag 300

gattactatc ctatggacta ctggggccaa gggaccacgg tcaccgtctc g 351

<210> 62
<211> 15
<212> DNA
<213> Mus musculus

<400> 62
aaatatgtta tacat 15

<210> 63
<211> 51
<212> DNA
<213> Mus musculus

<400> 63
tatattaatc cttacaatga tgttagtaag tacaatgaga agttcagagg c 51

<210> 64
<211> 27
<212> DNA
<213> Mus musculus

<400> 64
aataaggatt actatcctat ggactac 27

<210> 65
<211> 117
<212> PRT
<213> Homo sapiens

<400> 65

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Lys Tyr
            20                  25                  30

Val Ile His Trp Val Arg Gln Ala Pro Gly Gln Gly Leu Glu Trp Met
        35                  40                  45

Gly Tyr Ile Asn Pro Tyr Asn Asp Val Ser Lys Tyr Asn Glu Lys Phe
    50                  55                  60

Arg Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asn Lys Asp Tyr Tyr Pro Met Asp Tyr Trp Gly Gln Gly Thr
            100                 105                 110

Thr Val Thr Val Ser
        115
```

<210> 66
<211> 5
<212> PRT
<213> Mus musculus

<400> 66

```
Lys Tyr Val Ile His
1               5
```

<210> 67
<211> 17
<212> PRT

<213> Mus musculus

<400> 67

Tyr Ile Asn Pro Tyr Asn Asp Val Ser Lys Tyr Asn Glu Lys Phe Arg
1 5 10 15

Gly

<210> 68
<211> 9
<212> PRT
<213> Mus musculus

<400> 68

Asn Lys Asp Tyr Tyr Pro Met Asp Tyr
1 5

<210> 69
<211> 11
<212> PRT
<213> Mus musculus

<400> 69

Ser Ala Ser Ser Ser Val Arg Phe Ile His Trp
1 5 10

<210> 70
<211> 7
<212> PRT
<213> Mus musculus

<400> 70

Asp Thr Ser Lys Leu Ala Ser
1 5

<210> 71
<211> 9
<212> PRT
<213> Mus musculus

<400> 71

Gln Gln Trp Ser Ser Ser Pro Phe Thr
1 5

<210> 72
<211> 6
<212> PRT
<213> Mus musculus

<400> 72

Asp Tyr Tyr Ile His Trp
1 5

<210> 73
<211> 17
<212> PRT
<213> Mus musculus

<400> 73

Trp Ile Asp Pro Glu Asn Gly Asp Thr Glu Phe Val Pro Lys Phe Gln
1 5 10 15

Gly

<210> 74
<211> 4
<212> PRT
<213> Mus musculus

<400> 74

Thr Gly Gly Phe
1

<210> 75
<211> 10
<212> PRT
<213> Homo sapiens

<400> 75

Lys Pro Leu Pro Glu Val Thr Asp Glu Tyr
1 5 10

<210> 76
<211> 18
<212> PRT
<213> Homo sapiens

<400> 76

Glu Lys Glu Ala Leu Lys Lys Ile Ile Glu Asp Gln Gln Glu Ser Leu
1 5 10 15

Asn Lys

<210> 77
<211> 408
<212> PRT
<213> Homo sapiens

<400> 77

```
Met Ala Glu Asn Gly Asp Asn Glu Lys Met Ala Ala Leu Glu Ala Lys
1               5                   10                  15

Ile Cys His Gln Ile Glu Tyr Tyr Phe Gly Asp Phe Asn Leu Pro Arg
            20                  25                  30

Asp Lys Phe Leu Lys Glu Gln Ile Lys Leu Asp Glu Gly Trp Val Pro
        35                  40                  45

Leu Glu Ile Met Ile Lys Phe Asn Arg Leu Asn Arg Leu Thr Thr Asp
    50                  55                  60

Phe Asn Val Ile Val Glu Ala Leu Ser Lys Ser Lys Ala Glu Leu Met
65                  70                  75                  80

Glu Ile Ser Glu Asp Lys Thr Lys Ile Arg Arg Ser Pro Ser Lys Pro
                85                  90                  95

Leu Pro Glu Val Thr Asp Glu Tyr Lys Asn Asp Val Lys Asn Arg Ser
            100                 105                 110

Val Tyr Ile Lys Gly Phe Pro Thr Asp Ala Thr Leu Asp Asp Ile Lys
        115                 120                 125

Glu Trp Leu Glu Asp Lys Gly Gln Val Leu Asn Ile Gln Met Arg Arg
    130                 135                 140

Thr Leu His Lys Ala Phe Lys Gly Ser Ile Phe Val Val Phe Asp Ser
145                 150                 155                 160

Ile Glu Ser Ala Lys Lys Phe Val Glu Thr Pro Gly Gln Lys Tyr Lys
                165                 170                 175

Glu Thr Asp Leu Leu Ile Leu Phe Lys Asp Asp Tyr Phe Ala Lys Lys
            180                 185                 190

Asn Glu Glu Arg Lys Gln Asn Lys Val Glu Ala Lys Leu Arg Ala Lys
        195                 200                 205

Gln Glu Gln Glu Ala Lys Gln Lys Leu Glu Glu Asp Ala Glu Met Lys
    210                 215                 220

Ser Leu Glu Glu Lys Ile Gly Cys Leu Leu Lys Phe Ser Gly Asp Leu
225                 230                 235                 240
```

Asp Asp Gln Thr Cys Arg Glu Asp Leu His Ile Leu Phe Ser Asn His
245 250 255

Gly Glu Ile Lys Trp Ile Asp Phe Val Arg Gly Ala Lys Glu Gly Ile
260 265 270

Ile Leu Phe Lys Glu Lys Ala Lys Glu Ala Leu Gly Lys Ala Lys Asp
275 280 285

Ala Asn Asn Gly Asn Leu Gln Leu Arg Asn Lys Glu Val Thr Trp Glu
290 295 300

Val Leu Glu Gly Glu Val Glu Lys Glu Ala Leu Lys Lys Ile Ile Glu
305 310 315 320

Asp Gln Gln Glu Ser Leu Asn Lys Trp Lys Ser Lys Gly Arg Arg Phe
325 330 335

Lys Gly Lys Gly Lys Gly Asn Lys Ala Ala Gln Pro Gly Ser Gly Lys
340 345 350

Gly Lys Val Gln Phe Gln Gly Lys Lys Thr Lys Phe Ala Ser Asp Asp
355 360 365

Glu His Asp Glu His Asp Glu Asn Gly Ala Thr Gly Pro Val Lys Arg
370 375 380

Ala Arg Glu Glu Thr Asp Lys Glu Glu Pro Ala Ser Lys Gln Gln Lys
385 390 395 400

Thr Glu Asn Gly Ala Gly Asp Gln
405

<210> 78
<211> 17
<212> PRT
<213> Mus musculus

<400> 78

Lys Ser Ser Gln Ser Leu Leu Asn Ser Arg Thr Pro Lys Asn Tyr Leu
1 5 10 15

Ala

<210> 79
<211> 7
<212> PRT
<213> Mus musculus

<400> 79

Trp Ala Ser Thr Arg Lys Ser
1               5

<210> 80
<211> 8
<212> PRT
<213> Mus musculus

<400> 80

Lys Gln Ser Tyr Asn Leu Leu Thr
1               5

<210> 81
<211> 5
<212> PRT
<213> Mus musculus

<400> 81

His Tyr Tyr Ile Tyr
1               5

<210> 82
<211> 17
<212> PRT
<213> Mus musculus

<400> 82

Gly Val Asn Pro Ser Asn Gly Gly Thr His Phe Asn Glu Lys Phe Lys
1               5                   10                  15

Ser

<210> 83
<211> 11
<212> PRT
<213> Mus musculus

<400> 83

Ser Glu Tyr Asp Tyr Gly Leu Gly Phe Ala Tyr
1               5                   10

<210> 84
<211> 17
<212> PRT
<213> Mus musculus

<400> 84

Arg Ser Ser Gln Ser Leu Leu Asp Ser Arg Thr Arg Lys Asn Tyr Leu
1 5 10 15

Ala

<210> 85
<211> 7
<212> PRT
<213> Mus musculus

<400> 85

Trp Ala Ser Thr Arg Glu Ser
1 5

<210> 86
<211> 8
<212> PRT
<213> Mus musculus

<400> 86

Lys Gln Ser Tyr Asn Leu Pro Thr
1 5

<210> 87
<211> 5
<212> PRT
<213> Mus musculus

<400> 87

Asp Phe Trp Met Asn
1 5

<210> 88
<211> 19
<212> PRT
<213> Mus musculus

<400> 88

Gln Ile Arg Asn Lys Pro Asn Asn Tyr Glu Thr Tyr Tyr Ser Asp Ser
1 5 10 15

Leu Lys Gly

<210> 89
<211> 8
<212> PRT
<213> Mus musculus

<400> 89

Leu Gly Asn Ser Trp Phe Ala Tyr
1 5

<210> 90
<211> 34
<212> DNA
<213> Artificial Sequence

<220>
<223> Primer

<220>
<221> misc_feature
<222> (17)..(17)
<223> n is a, c, g, or t

<400> 90
tttttggatc csargtnmag ctgsagsagt cwgg 34

<210> 91
<211> 30
<212> DNA
<213> Artificial Sequence

<220>
<223> Primer

<400> 91
ggaagatcta tagacagatg ggggtgtcgt 30

<210> 92
<211> 36
<212> DNA
<213> Artificial Sequence

<220>
<223> Primer

<400> 92
tttttgaatt ctgayattgt gmtsacmcar wctmca 36

<210> 93
<211> 32
<212> DNA
<213> Artificial Sequence

<220>
<223> Primer

<400> 93
tttttgggcc cggatacagt tggtgcagca tc 32

<210> 94
<211> 51
<212> DNA
<213> Artificial Sequence

<220>
<223> Primer

<400> 94

ggcccagccg gccatggcgg actacaaaga agtacagctg caggagtcag g 51

<210> 95
<211> 50
<212> DNA
<213> Artificial Sequence

<220>
<223> Primer

<400> 95
ggagccgccg ccgccagaac caccaccacc tgaggagacg gtgaccgtgg 50

<210> 96
<211> 53
<212> DNA
<213> Artificial Sequence

<220>
<223> Primer

<400> 96
ggcggcggcg gctccggtgg tggtggatcc gatattgtga tgacacagtc tac 53

<210> 97
<211> 29
<212> DNA
<213> Artificial Sequence

<220>
<223> Primer

<400> 97
gcggccgcgg atacagttgg tgcagcatc 29

<210> 98
<211> 39
<212> DNA
<213> Artificial Sequence

<220>
<223> Primer

<400> 98
gggcccgaat tcatggcgga ctacaaagag gtgcagctg 39

<210> 99
<211> 39
<212> DNA
<213> Artificial Sequence

<220>
<223> Primer

<400> 99
gggcccaagc ttggatacag ttggtgcagc atcagcccg 39

<210> 100

<211> 100
<212> DNA
<213> Artificial Sequence

<220>
<223> Oligonukleotid

<400> 100

caggttcagc tggtgcagtc tggagctgag gtgaagaagc ctggggcctc agtgaaggtc 60

tcctgtaagg cttctggtta cacctttacc aaatatgtta 100

<210> 101
<211> 100
<212> DNA
<213> Artificial Sequence

<220>
<223> Oligonukleotid

<400> 101

acatcattgt aaggattaat atatcccatc cactcaagcc cttgtccagg ggcctgtcgc 60

acccaatgta taacatattt ggtaaaggtg taaccagaag 100

<210> 102
<211> 100
<212> DNA
<213> Artificial Sequence

<220>
<223> Oligonukleotid

<400> 102

gatgggatat attaatcctt acaatgatgt tagtaagtac aatgagaagt tcagaggcag 60

agtcaccatg accacagaca catccacgag cacagcctac 100

<210> 103
<211> 100
<212> DNA
<213> Artificial Sequence

<220>
<223> Oligonukleotid

<400> 103

gatagtaatc cttatttctc gcacagtaat acacggccgt gtcgtcagat ctcaggctcc 60

tcagctccat gtaggctgtg ctcgtggatg tgtctgtggt 100

<210> 104
<211> 86
<212> DNA
<213> Artificial Sequence

<220>
<223> Oligonukleotid

<400> 104

attactgtgc gagaaataag gattactatc ctatggacta ctggggccaa gggaccacgg 60

tcaccgtctc gggaggagga ggatcc 86

<210> 105
<211> 37
<212> DNA
<213> Artificial Sequence

<220>
<223> Primer

<400> 105
ggcccagccg gcccaggttc agctggtgca gtctgga 37

<210> 106
<211> 39
<212> DNA
<213> Artificial Sequence

<220>
<223> Primer

<400> 106
ggatcctcct cctcccgaga cggtgaccgt ggtcccttg 39

<210> 107
<211> 90
<212> DNA
<213> Artificial Sequence

<220>
<223> Oligonukleotid

<400> 107

gaaattgtgt tgacacagtc tccagccacc ctgtctttgt ctccagggga aagagccacc 60

ctctcctgca gtgccagttc aagtgtaagt 90

<210> 108
<211> 91
<212> DNA
<213> Artificial Sequence

<220>

<223> Oligonukleotid

<400> 108

tgtgtcatag atgaggagcc tgggagcctg gccaggtttc tgttggtacc agtgcatgta 60

acttacactt gaactggcac tgcaggagag g 91

<210> 109
<211> 91
<212> DNA
<213> Artificial Sequence

<220>
<223> Oligonukleotid

<400> 109

ccaggctccc aggctcctca tctatgacac atccaaactg gcttctggca tcccagccag 60

gttcagtggc agtgggtctg ggacagactt c 91

<210> 110
<211> 91
<212> DNA
<213> Artificial Sequence

<220>
<223> Oligonukleotid

<400> 110

ccactgctga cagtaataaa ctgcaaaatc ttcaggctct aggctgctga tggtgagagt 60

gaagtctgtc ccagacccac tgccactgaa c 91

<210> 111
<211> 79
<212> DNA
<213> Artificial Sequence

<220>
<223> Oligonukleotid

<400> 111

agattttgca gtttattact gtcagcagtg gagtagtaac ccgctcacgt tcggcggagg 60

gaccaaggtg gagatcaaa 79

<210> 112
<211> 27
<212> DNA
<213> Artificial Sequence

<220>
<223> Primer

<400> 112
ggatccgaaa ttgtgttgac acagtct 27

<210> 113
<211> 29
<212> DNA
<213> Artificial Sequence

<220>
<223> Primer

<400> 113
gcggccgctt tgatctccac cttggtccc 29

<210> 114
<211> 50
<212> DNA
<213> Artificial Sequence

<220>
<223> Primer

<400> 114
ggatccgccg ccgccagaac caccaccacc ggagcctcct cctcccgaga 50

<210> 115
<211> 100
<212> DNA
<213> Artificial Sequence

<220>
<223> Oligonukleotid

<400> 115

**gatatccaga tgactcaaag tcctagttcc ctgtctgcat cagtgggaga ccgggtgacc 60**

**attacatgcg gtacatccca agacatcaat aattatctca 100**

<210> 116
<211> 100
<212> DNA
<213> Artificial Sequence

<220>
<223> Oligonukleotid

<400> 116

**cggaatgcag cctggatgtg tagtagatta ataacttagg aactttgcct ggcttctgct 60**

**gataccagtt gagataatta ttgatgtctt gggatgtacc 100**

<210> 117
<211> 100
<212> DNA
<213> Artificial Sequence

<220>
<223> Oligonukleotid

<400> 117

**aatctactac acatccaggc tgcattccgg ggtgccctcc cgcttttcgg gctccgggtc 60**

**gggaaccgac tttaccctaa ccatatcttc cctgcagcct 100**

<210> 118
<211> 111
<212> DNA
<213> Artificial Sequence

<220>
<223> Oligonukleotid

<400> 118

**aagtacagtg agttgcgtcc caccaccaaa tgtccatggt aatgtctttg actgctgaca 60**

**atagtacgtt gcaacgtctt caggctgcag ggaagatatg gttagggtaa a 111**

<210> 119
<211> 29
<212> DNA
<213> Artificial Sequence

<220>
<223> Primer

<400> 119
ctcgaggaga tatccagatg actcaaagt 29

<210> 120
<211> 57
<212> DNA
<213> Artificial Sequence

<220>
<223> Primer

<400> 120
agcggcactt gctccgctcc ctcctccacc actgccacct ccaccaagta cagtgag 57

<210> 121
<211> 100
<212> DNA
<213> Artificial Sequence

<220>

<223> Oligonukleotid

<400> 121

caggtgcagc tagtggagtc cggtggcggc ctcgttaagc cgggcggatc gctgcgcctt 60

tcatgtgccg catcaggatt cacattctcc agttactcta 100

<210> 122
<211> 100
<212> DNA
<213> Artificial Sequence

<220>
<223> Oligonukleotid

<400> 122

cttccacctg aatcattaat gtacgagacc cattccaatc ccttgccagg tgcctgccga 60

atccatgaca tagagtaact ggagaatgtg aatcctgatg 100

<210> 123
<211> 100
<212> DNA
<213> Artificial Sequence

<220>
<223> Oligonukleotid

<400> 123

ggtctcgtac attaatgatt caggtggaag tacattctat ccggacacgg ttaaaggtag 60

atttaccatc agccgtgata acgcgaagaa tagcttgtac 100

<210> 124
<211> 100
<212> DNA
<213> Artificial Sequence

<220>
<223> Oligonukleotid

<400> 124

tgccataata catacgtcga gcgcaataat atacagcagt atcctctgca cgcaggctat 60

tcatctgtaa gtacaagcta ttcttcgcgt tatcacggct 100

<210> 125
<211> 86
<212> DNA

<213> Artificial Sequence

<220>
<223> Oligonukleotid

<400> 125

attattgcgc tcgacgtatg tattatggca atagtcactg gcactttgac gtctggggcc 60

agggcacgac agttactgtc tcttcg 86

<210> 126
<211> 58
<212> DNA
<213> Artificial Sequence

<220>
<223> Primer

<400> 126
ggagcaagtg ccgctggagg cggaggttca ggcggtggtg gaagccaggt gcagctag 58

<210> 127
<211> 27
<212> DNA
<213> Artificial Sequence

<220>
<223> Primer

<400> 127
gggccccgaa gagacagtaa ctgtcgt 27

<210> 128
<211> 29
<212> DNA
<213> Artificial Sequence

<220>
<223> Primer

<400> 128
ctcgaggaga tatccagatg actcaaagt 29

<210> 129
<211> 27
<212> DNA
<213> Artificial Sequence

<220>
<223> Primer

<400> 129
gggccccgaa gagacagtaa ctgtcgt 27

<210> 130
<211> 25

<212> PRT
<213> Artificial Sequence

<220>
<223> Peptid

<400> 130

```
Gly Gly Gly Gly Ser Gly Gly Gly Gly Ser Gly Ala Ser Ala Ala Gly
1               5                   10                  15

Gly Gly Gly Ser Gly Gly Gly Gly Ser
            20                  25
```

<210> 131
<211> 15
<212> PRT
<213> Artificial Sequence

<220>
<223> Peptid

<400> 131

```
Gly Gly Gly Gly Ser Gly Gly Gly Gly Ser Gly Gly Gly Gly Ser
1               5                   10                  15
```

<210> 132
<211> 5
<212> PRT
<213> Artificial Sequence

<220>
<223> Peptid

<400> 132

```
Gly Gly Gly Gly Ser
1               5
```

<210> 133
<211> 5
<212> PRT
<213> Artificial Sequence

<220>
<223> Peptid

<400> 133

```
Ala Ala Ala Arg Gly
1               5
```

<210> 134
<211> 40
<212> DNA
<213> Artificial Sequence

<220>
<223> Primer

<400> 134
ggcccagccg gccggatccg atatccagat gactcaaagt 40

<210> 135
<211> 41
<212> DNA
<213> Artificial Sequence

<220>
<223> Primer

<400> 135
gcggccgcgg atcctcctcc tcccgaagag acagtaactg t 41

<210> 136
<211> 39
<212> DNA
<213> Artificial Sequence

<220>
<223> Oligonukleotid

<400> 136
ggccgctaaa cccctacctg aagtgactga tgagtatgc 39

<210> 137
<211> 39
<212> DNA
<213> Artificial Sequence

<220>
<223> Oligonukleotid

<400> 137
tcgagcatac tcatcagtca cttcaggtag gggtttagc 39

<210> 138
<211> 100
<212> DNA
<213> Artificial Sequence

<220>
<223> Oligonukleotid

<400> 138

**caagtgcaac tggtccagtc aggtgctgag gtgaaaaaac ccggagccag tgtcaaagta 60**

**agctgcaagg cctctgggta tactttcacc cattactata 100**

<210> 139
<211> 100
<212> DNA
<213> Artificial Sequence

<220>
<223> Oligonukleotid

<400> 139

ccaccgttcg aagggttgac tccacccatc cactcaagcc cctgacctgg agcttgacga 60

acccagtata tatagtaatg ggtgaaagta tacccagagg 100

<210> 140
<211> 100
<212> DNA
<213> Artificial Sequence

<220>
<223> Oligonukleotid

<400> 140

gatgggtgga gtcaacccctt cgaacggtgg cactcacttc aatgaaaagt ttaaaagccg 60

cgtaaccatg acgcgagata cttccatttc cacagcttat 100

<210> 141
<211> 100
<212> DNA
<213> Artificial Sequence

<220>
<223> Oligonukleotid

<400> 141

cataatcata ttcacttcta gcacagtaat aaacggccgt gtcatcactg cgtaacctac 60

taagttccat ataagctgtg gaaatggaag tatctcgcgt 100

<210> 142
<211> 80
<212> DNA
<213> Artificial Sequence

<220>
<223> Oligonukleotid

<400> 142

attactgtgc tagaagtgaa tatgattatg ggttgggttt cgcttactgg ggccagggaa 60

ccctcgtcac cgtgtccagt 80

<210> 143
<211> 33
<212> DNA

<213> Artificial Sequence

<220>
<223> Primer

<400> 143
ctcgaggaca agtgcaactg gtccagtcag gtg 33

<210> 144
<211> 55
<212> DNA
<213> Artificial Sequence

<220>
<223> Primer

<400> 144
agaaccgcca ccaccggatc ctccaccacc actggacacg gtgacgaggg ttccc 55

<210> 145
<211> 100
<212> DNA
<213> Artificial Sequence

<220>
<223> Oligonukleotid

<400> 145

gacattgtta tgacgcagag ccctgattca ctcgcagtgt ccctaggaga gcgggccacc 60

atcaactgta aaagttctca gtccctgctg aacagcagga 100

<210> 146
<211> 100
<212> DNA
<213> Artificial Sequence

<220>
<223> Oligonukleotid

<400> 146

gcccagtaaa tgagcagctt aggcggctgt ccaggtttct gttggtacca tgccaggtaa 60

ttcttaggcg tcctgctgtt cagcagggac tgagaacttt 100

<210> 147
<211> 100
<212> DNA
<213> Artificial Sequence

<220>
<223> Oligonukleotid

<400> 147

acagccgcct aagctgctca tttactgggc ctccacacgg aagagcggcg tgcccgaccg 60

gttttccggg agcggctccg gcaccgactt taccttgacc 100

<210> 148
<211> 100
<212> DNA
<213> Artificial Sequence

<220>
<223> Oligonukleotid

<400> 148

caaatgtcag gagattgtaa gattgcttgc aatagtatac ggccacgtct tctgcctgca 60

gggaactgat ggtcaaggta aagtcggtgc cggagccgct 100

<210> 149
<211> 56
<212> DNA
<213> Artificial Sequence

<220>
<223> Oligonukleotid

<400> 149
gcaagcaatc ttacaatctc ctgacatttg gcggcggcac aaaagtggag atcaaa 56

<210> 150
<211> 55
<212> DNA
<213> Artificial Sequence

<220>
<223> Primer

<400> 150
ggtggtggcg gttctggcgg aggcggatcc gacattgtta tgacgcagag ccctg 55

<210> 151
<211> 31
<212> DNA
<213> Artificial Sequence

<220>
<223> Primer

<400> 151
gggccctttg atctccactt ttgtgccgcc g 31

<210> 152
<211> 33
<212> DNA
<213> Artificial Sequence

<220>
<223> Primer

<400> 152
ctcgaggaca agtgcaactg gtccagtcag gtg 33

<210> 153
<211> 31
<212> DNA
<213> Artificial Sequence

<220>
<223> Primer

<400> 153
gggcccttg atctccactt ttgtgccgcc g 31

<210> 154
<211> 72
<212> DNA
<213> Artificial Sequence

<220>
<223> Oligonukleotid

<400> 154

ggccgctctg gagaaagaag cactgaagaa aataatagaa gaccaacaag aatccctaaa 60

caaagcagct gc 72

<210> 155
<211> 72
<212> DNA
<213> Artificial Sequence

<220>
<223> Oligonukleotid

<400> 155

tcgagcagct gctttgttta gggattcttg ttggtcttct attattttct tcagtgcttc 60

tttctccaga gc 72

<210> 156
<211> 100
<212> DNA
<213> Artificial Sequence

<220>
<223> Oligonukleotid

<400> 156

gaggtgcaac tggtcgaaag tggcggtggt ttagttcagc ctggtggaag tctacggctt 60

agctgcgcag catccggttt cacctttagc gacttttgga 100

<210> 157
<211> 100
<212> DNA
<213> Artificial Sequence

<220>
<223> Oligonukleotid

<400> 157

ttattcggtt tgttgcggat ttgcccaacc cactccagtc ctttgcccgg agcctgccga 60

acccagttca tccaaaagtc gctaaaggtg aaaccggatg 100

<210> 158
<211> 100
<212> DNA
<213> Artificial Sequence

<220>
<223> Oligonukleotid

<400> 158

ggttgggcaa atccgcaaca aaccgaataa ctacgaaact tattactcag atagcctgaa 60

gggtcgattc accatcagca gggatgattc aaagtcaatc 100

<210> 159
<211> 100
<212> DNA
<213> Artificial Sequence

<220>
<223> Oligonukleotid

<400> 159

aggagttacc tagtgtacag taatacaccg cagtatcctc cgctcttaat gagttcatct 60

gtaggtaagt gattgacttt gaatcatccc tgctgatggt 100

<210> 160
<211> 74
<212> DNA
<213> Artificial Sequence

<220>
<223> Oligonukleotid

<400> 160

cggtgtatta ctgtacacta ggtaactcct ggttcgcgta ttggggacag ggcacccttg 60

taaccgtctc cagc 74

<210> 161
<211> 65
<212> DNA
<213> Artificial Sequence

<220>
<223> Primer

<400> 161

ggtggcggcg gctccggagg tggtggttcc ggcggtggcg gatcggaggt gcaactggtc 60

gaaag 65

<210> 162
<211> 27
<212> DNA
<213> Artificial Sequence

<220>
<223> Primer

<400> 162
gggcccgctg gagacggtta caagggt 27

<210> 163
<211> 100
<212> DNA
<213> Artificial Sequence

<220>
<223> Oligonukleotid

<400> 163

gacattgtta tgacccagag cccggactct ctcgctgtta gtcttggtga gcgagcgact 60

attaactgcc ggagcagtca gagtttgttg gactcacgga 100

<210> 164
<211> 100
<212> DNA
<213> Artificial Sequence

<220>
<223> Oligonukleotid

<400> 164

gcccaatata tcagtaattt aggtggttgg cccggcttct gctggtacca tgccaggtag 60

ttctttctcg tccgtgagtc caacaaactc tgactgctcc 100

<210> 165
<211> 100
<212> DNA
<213> Artificial Sequence

<220>
<223> Oligonukleotid

<400> 165

ccaaccacct aaattactga tatattgggc gtcgactcgt gagtcagggg taccggacag 60

gttttctgga agcggatcag gaacagactt cactttgacg 100

<210> 166
<211> 100
<212> DNA
<213> Artificial Sequence

<220>
<223> Oligonukleotid

<400> 166

caaatgtcgg cagattatag ctttgcttac aataataaac cgcaacgtcc tcggcttgaa 60

gcgaagagat cgtcaaagtg aagtctgttc ctgatccgct 100

<210> 167
<211> 56
<212> DNA
<213> Artificial Sequence

<220>
<223> Oligonukleotid

<400> 167
gtaagcaaag ctataatctg ccgacatttg gtggcggcac caaggttgaa attaag 56

<210> 168
<211> 29
<212> DNA
<213> Artificial Sequence

<220>
<223> Primer

<400> 168
ctcgaggaga cattgttatg acccagagc 29

<210> 169
<211> 65
<212> DNA
<213> Artificial Sequence

<220>

<223> Primer

<400> 169

ggagccgccg ccaccggaac cgccgccgcc agatcctccg ccgcccttaa tttcaacctt 60

ggtgc 65

<210> 170
<211> 29
<212> DNA
<213> Artificial Sequence

<220>
<223> Primer

<400> 170
ctcgaggaga cattgttatg acccagagc 29

<210> 171
<211> 27
<212> DNA
<213> Artificial Sequence

<220>
<223> Primer

<400> 171
gggcccgctg gagacggtta caagggt 27

<210> 172
<211> 5
<212> PRT
<213> Artificial Sequence

<220>
<223> Peptid

<400> 172

Met Ala Asp Tyr Lys
1 5

## Beschreibung

Die Erfindung betrifft rekombinante an Prostata-spezifisches Stammzellantigen (PSCA) bindende Antikörper und deren Verwendung in der Diagnostik und Therapie. Die Antikörper eignen sich zur Anwendung im Bereich der Medizin, Pharmazie und der biomedizinischen Forschung.

Das Prostatakarzinom ist eine der häufigsten krebsbedingten Todesursachen in Deutschland. Die Standardtherapie bei einem primären, organbegrenzten Prostatakarzinom ist derzeit die radikale Prostatektomie, d. h. die vollständige Entfernung von Prostata, Samenblasen und Lymphknoten. Eine Alternative dazu stellt die Strahlentherapie dar, die durch Spickung der Prostata mit radioaktivem Material (Brachytherapie) erfolgt. Die meisten Patienten mit primärem, lokalem Prostatakarzinom können erfolgreich durch radikale Prostatektomie und Strahlentherapie behandelt werden. Eine Rezidivbildung tritt bei etwa 20 - 40 % der Betroffenen auf [Roehl 2004].

Gegenwärtig werden verschiedene antikörperbasierte Therapieverfahren entwickelt. Dabei ist es von entscheidender Bedeutung, Zielstrukturen auf den Krebszellen zu identifizieren, die als Angriffsstellen für die antikörperbasierten Therapeutika dienen können. Eine geeignete Zielstruktur als Angriffsstelle für die Behandlung von Prostatakrebs sind Oberflächenproteine, die hauptsächlich im Prostatagewebe vorliegen und die eine Überexpression auf malignen im Vergleich zu gesunden Zellen zeigen.

Das Prostata-spezifische Stammzellantigen (PSCA) ist ein derartiges Oberflächenmolekül, das spezifisch auf Prostatazellen vorliegt und das in Prostatakarzinomzellen im Vergleich zum gesunden Gewebe vermehrt exprimiert wird (tumorassoziiertes Antigen). Humanes PSCA umfasst 123 Aminosäuren und weist eine N-terminale Signalsequenz, eine C-terminale Glykosylphosphatidylinositol(GPI)-Verankerungssequenz und mehrere N-Glykosylierungsstellen auf. PSCA wird zur Thy-1/Ly-6 Familie der GPI-verankerten Zelloberflächenmoleküle gezählt, da es u.a. die für diese Familie charakteristischen konservierten Cystein-Reste aufweist [Reiter 1998].

Aufgrund der erhöhten Expression in Prostatatumoren ist PSCA ein geeignetes Zielmolekül für die Therapie von Prostatakrebs und es wurden bislang verschiedene Therapiestrategien, die auf PSCA als Target abzielen, entwickelt. Ein Nachweis der Wirksamkeit von PSCA als therapeutischem Target bei der Krebstherapie am Menschen wurde u.a. durch die Vakzinierung mit dendritischen Zellen, die zuvor mit einem PSCA-Peptid beladen wurden, erbracht. Klinische Studien (Phase 1 und 2) mit 12 Hormon- und Chemotherapie-refraktären Prostatakarzinom-Patienten zeigten, dass eine Vakzinierung mit PSCA-Peptid-beladenen Dendritischen Zellen bei fünf der Patienten eine T-Zell-Antwort gegen PSCA verursachte, die im Mittel mit einer verlängerten Überlebenszeit korrelierte. Bei einem Patienten konnte sogar eine Reduktion der Tumormasse beobachtet werden [Thomas-Kaskel 2006]. WO 2009/032949 A2 offenbart monoklonale anti-PSCA-Antikörper (1G8), die zum Targeting von Tumoren und zu deren Detektion eingesetzt werden. Die CDR-Regionen der Antikörper haben die folgenden Aminosäuresequenzen:

| | variable Region der leichten Kette (1G8) | | variable Region der schweren Kette (1G8) | |
|---|---|---|---|---|
| CDR1 | SASSSVRFIHW | SEQ ID No. 69 | DYYIHW | SEQ ID No. 72 |
| CDR2 | DTSKLAS | SEQ ID No. 70 | WIDPENGDTEFVPKFQG | SEQ ID No. 73 |
| CDR3 | QQWSSSPFT | SEQ ID No. 71 | TGGF | SEQ ID No. 74 |

Aufgrund der zytotoxischen Eigenschaften des 1G8 Antikörpers ist dieser für Targetingstrategien, die auf der Rekrutierung von Effektorzellen beruhen, ungeeignet (Gu 2005).

Die in WO 2009/032949 A2 offenbarten Antikörper werden diagnostisch bevorzugt in Form von funktionellen murinen und humanisierten monoklonalen Antikörpern sowie mit Radionukliden markierten PSCA-spezifischen Minibodies und Diabodies eingesetzt.

[Feldmann 2010] entwickelten bispezifische rekombinante Antikörper mit einem PSCA-bindenden Paratop und einem CD3-bindenden Paratop. Das PSCA-bindende Paratop der bispezifischen Antikörper ist vom PSCA-Antikörper 7F5 abgeleitet [Morgenroth 2007] und umfasst CDR-Regionen mit den folgenden Aminosäuresequenzen:

| | variable Region der leichten Kette (7F5) | | variable Region der schweren Kette (7F5) | |
|---|---|---|---|---|
| CDR1 | RTSQDISNYLN | SEQ ID No. 27 | SYTMS | SEQ ID No. 30 |
| CDR2 | YTLKLNS | SEQ ID No. 28 | YIHNGGGHTYYPDTIKG | SEQ ID No. 31 |
| CDR3 | QQSKTLPWT | SEQ ID No. 29 | RMYYGNSHWYFDV | SEQ ID No. 32 |

Die in [Feldmann 2010] offenbarten bispezifischen Antikörper führten *in vitro* erfolgreich zur spezifischen T-Zell-vermittelte Lyse PSCA-positiver Tumorzellen. Für eine spezifische Lyse von etwa 40 % der eingesetzten PSCA-positiven Tumorzellen ist bei einem Verhältnis von Effektorzellen zu PSCA-positiven Tumorzellen von 20:1 mindestens 5 ng der in [Feldmann 2010] offenbarten bispezifischen Antikörper einzusetzen. In vitro konnte mit dem in [Feldmann 2010] offenbarten Antikörper eine spezifische Lyse von maximal etwa 60 % der eingesetzten PSCA-positiven Tumorzellen erreicht werden (in Gegenwart von 50 - 100 ng des Antikörpers). Eine weitere Erhöhung der Effektivität konnte nicht nachgewiesen werden. Auch in Gegenwart von 1000 ng des bispezifischen Antikörpers konnte kein höherer Anteil PSCA-positiver Tumorzellen lysiert werden.

Die bekannten *in vitro* und *in vivo* Daten zeigen, dass PSCA ein großes Potenzial als Zielantigen für die Immuntherapie von Prostatakarzinomen besitzt und als diagnostisches Target geeignet ist. Aufgabe der Erfindung ist es, verbesserte Antikörper gegen PSCA bereitzustellen, die insbesondere in Form von bispezifischen rekombinanten Antikörpern in geringer Konzentration therapeutisch wirksam sind und PSCA-positive Tumorzellen effektiv lysieren können.

Die Aufgabe wird erfindungsgemäß gelöst durch einen an Prostata-spezifisches Stammzellantigen (PSCA) bindenden Antikörper (hierin auch als anti-PSCA Antikörper bezeichnet), der komplementaritätsbestimmende Regionen (CDR) mit den folgenden Aminosäure-Sequenzen enthält:

- CDR der variablen Region der leichten Kette: CDR1 SEQ ID No. 1, CDR2 SEQ ID No. 2, CDR3 SEQ ID No. 3 und

- CDR der variablen Region der schweren Kette: CDR1 SEQ ID No. 4, CDR2 SEQ ID No. 5, CDR3 SEQ ID No. 6.

Der erfindungsgemäße Antikörper, der durch die vorganannten CDRs gekennzeichnet ist, wird hierin auch vereinfacht als MB1 bezeichnet. Die Erfinder stellten im Rahmen umfangreicher Untersuchungen überraschend fest, dass der erfindungsgemäße Antikörper in Form von rekombinant hergestellten bispezifischen Antikörpern die spezifische Lyse PSCA-positiver Tumorzellen auch in sehr geringen Mengen vermitteln kann. Ferner wurde überraschend festgestellt, dass die durch die neuen Antikörper vermittelte spezifische Lyse von PSCA-positiven Tumorzellen effektiver ist. So konnten in *in vitro* Untersuchungen mit bispezifischen Antikörpern, die einen erfindungsgemäßen anti-PSCA Antikörper enthalten über 90 % der eingesetzten PSCA-positiven Tumorzellen lysiert werden. Es kann mit dem neuen anti-PSCA Antikörper somit die eingesetzte Antikörpermenge deutlich reduziert werden. Gleichzeitig wird die therapeutische Wirksamkeit erhöht, da mit dem erfindungsgemäßen Antikörper PSCA-postive Tumorzellen effektiver lysiert werden können. Aufgrund der geringen Konzentration, in der mit dem erfindungsgemäßen Antikörper eine Wirksamkeit erzielt werden kann, ist mit dem erfindungsgemäßen auch eine verbesserte Abtötung von metastasierenden PSCA-positiven Zellen möglich. Vergleichsuntersuchungen mit dem aus dem Stand der Technik bekannten anti-PSCA 7F5 in einem vergleichbaren bispezifischen Konstrukt bestätigen deutlich die Überlegenheit des erfindungsgemäßen Antikörpers sowohl in *in vitro* (Fig. 3) als auch *in vivo* Studien (Fig. 4).

Bevorzugte erfindungsgemäße anti-PSCA Antikörper enthalten die wie vorstehend definierten CDR-Regionen, wobei die Aminosäuresequenz der variablen Regionen der leichten und schweren Kette mindestens 80 %, bevorzugt mindestens 90 %, besonders bevorzugt mindestens 95% Aminosäure-Sequenzidentität zu folgenden Aminosäuresequenz aufweist:

- variable Region der leichten Kette SEQ ID No. 24, variable Region der schweren Kette SEQ ID No. 26 oder
- variable Region der leichten Kette SEQ ID No. 20, variable Region der schweren Kette SEQ ID No. 22.

Bevorzugt sind davon anti-PSCA Antikörper mit den oben definierten CDR-Regionen, dessen variable Regionen eine humanisierte Struktur aufweisen. Besonders bevorzugt sind anti-PSCA Antikörper, deren variable Region der leichten Kette die Aminosäuresequenz gemäß SEQ ID No. 24 und deren variable Region der schweren Kette die Aminosäuresequenz gemäß SEQ ID No. 26 enthält.

Der Begriff "Antikörper" im erfindungsgemäßen Sinn umfasst alle Antikörper oder Antikörperfragmente, die in der Lage sind, spezifisch an ein Antigen zu binden. Bei rekombinanten Antikörpern handelt es sich dabei um Antikörper, die mit Hilfe von gentechnisch veränderten Organismen hergestellt werden. Der Begriff Antikörper umfasst sowohl die kompletten monoklonalen Antikörper als auch deren epitopbindende Fragmente. Hierbei umfassen die epitopbindenden Fragmente (hier auch als Antikörperfragmente bezeichnet) diejenigen Teile des Antikörpers, die in der Lage sind, an das Antigen zu binden. Antikörperfragmente im Sinne der Erfindung beinhalten Fab, Fab', $F(ab')_2$, Fd, Einzelketten (single-chain) variable Fragmente (scFv), Einzelketten-Antikörper, disulfidverlinkte variable Fragmente (sdFv) und Fragmente die entweder eine variable Region der leichten Kette ($V_L$) oder eine variable Region der schweren Kette ($V_H$) enthalten. Antikörperfragmente enthalten die variablen Regionen entweder allein oder in Kombination mit weiteren Regionen, die ausgewählt sind aus der Hinge-Region, und dem ersten, zweiten und dritten Bereich der konstanten Region ($C_H1$, $C_H2$, $C_H3$).

Weiterhin umfasst der Begriff Antikörper rekombinant hergestellte Antikörper, wie Diabodies, Triabodies und Tetrabodies. Ebenfalls vom Begriff Antikörper umfasst sind chimäre Antikörper, bei denen unterschiedliche Teile des Antikörpers aus verschiedenen Spezies stammen, wie beispielsweise Antikörper mit einer murinen variablen Region, welche mit einer humanen konstanten Region kombiniert ist. Humanisierte Antikörper sind hierin ebenfalls durch den Begriff Antikörper umfasst. Das Ziel der Humanisierung von Antikörpern liegt in der Reduktion der Immunogenität eines xenogenen Antikörpers, wie beispielsweise muriner Antikörper, zur Verwendung im humanen System, wobei die volle Bindungsaffinität und die Antigenspezifität erhalten bleiben. Humanisierte Antikörper können auf verschiedenen bekannten Wegen hergestellt werden, wie beispielsweise durch Resurfacing und CDR-Grafting. Beim Resurfacing werden durch eine Kombination aus Molecular Modelling, statistischen Analysen und Mutagenese alle nicht-CDR-Regionen auf der Oberfläche des Antikörpers verändert, so dass diese der Oberfläche von Antikörpern des Zielorganismus ähneln. Beim CDR-Grafting werden die CDR-Regionen des zu humanisierenden Antikörpers in humane variable Regionen eingebracht.

Antikörperfragmente sind gegebenenfalls untereinander über einen Linker verknüpft. Der Linker umfasst eine kurze (bevorzugt 10 bis 50 Aminosäurereste lange) Peptidsequenz, die so ausgewählt wird, dass das Antikörperfragment eine derartige dreidimensionale Faltung der $V_L$ und $V_H$ besitzt, dass es die Antigenspezifität des kompletten Antikörpers aufweist. Bevorzugt sind Glycin-Serin-Linker oder Linkerpeptide mit einer Aminosäuresequenz gemäß SEQ ID No. 75 oder SEQ ID No. 76.

Die Bezeichnung "variable Region" bedeutet hierin die Teile der schweren und leichten Ketten der Antikörper, die sich in ihrer Sequenz zwischen Antikörpern unterscheiden und die Spezifität des Antikörpers und die Bindung an sein Antigen bestimmen. Die Variabilität ist hierbei nicht gleichmäßig in der variablen Region verteilt, sondern ist üblicherweise innerhalb dreier definierter Segmente der variablen Region konzentriert, den komplementaritätsbestimmenden Regionen (CDRs, auch als hypervariable Regionen bezeichnet), die in den variablen Regionen sowohl der leichten als auch der schweren Kette enthalten sind. Die Antigenbindungsstelle eines Antikörpers, das sogenannte Paratop, ist durch die hypervariablen Regionen (CDR) der leichten und schweren Ketten des Antikörpers charakterisiert.

Zur Beschreibung von Antikörpern wird hierin auch die vereinfachte Bezeichnung anti-"Antigen" Antikörper verwendet, um darzustellen, dass es sich dabei um einen Antikörper handelt, der spezifisch an das in der Bezeichnung angegebene Antigen bindet. So ist beispielsweise unter einem "anti-PSCA Antikörper" im Sinn der Erfindung ein Antikörper zu verstehen, der spezifisch an das Antigen PSCA bindet. Unter einer spezifischen Bindung eines Antikörpers an ein bestimmtes Antigen wird hierin verstanden, dass ein Antikörper mit einer hohen Affinität an das bestimmte Antigen bindet und mit einer deutlich geringeren Affinität und vorzugsweise nicht an andere Antigene bindet.

Bevorzugte Antikörper liegen in Form eines scFv-Fragments oder eines F(ab')2-Fragments vor. Weiter bevorzugte Antikörper sind bispezifische Antikörper, die rekombinant hergestellt werden und die zwei unterschiedliche Paratope enthalten, von denen eines gegen PSCA und ein anderes nicht gegen PSCA gerichtet ist. Das andere Paratop des bispezifischen Antikörpers ist dabei vorzugsweise gegen eine Oberflächenstruktur auf einer Effektorzelle oder gegen ein 10 bis 50 Aminosäuren langes Peptid (bevorzugt eine 10 bis 50 Aminosäuren lange Sequenz des humanen La-Proteins, besonders bevorzugt gegen ein Peptid mit einer der Aminosäuresequenzen gemäß SEQ ID No. 75 oder SEQ ID No. 76) gerichtet.

Bevorzugt sind erfindungsgemäße anti-PSCA Antikörper (naive oder rekombinante Antikörper) mit einer Effektorgruppe konjugiert. Konjugation bedeutet hierbei die Ankopplung eines Stoffs, also der Effektorgruppe, an den Antikörper. Die Verbindung des Antikörpers zur Effektorgruppe wird bevorzugt durch rekombinante Expression in Form eines Fusionsproteins oder durch *in vitro* Methoden hergestellt, wobei die Effektorgruppe bevorzugt über chemische Linkergruppen an den Antikörper gebunden wird (beispielsweise über Thioetherbindungen oder Disulfidbindungen). Sie können an den Antikörper auch durch ein intermediäres Trägermolekül, beispielsweise Serumalbumin, gebunden sein. Gegebenenfalls enthält ein erfindungsgemäßer Antikörper mehrere Effektorgruppen. Effektorgruppen sind dabei vorzugsweise ausgewählt aus Wirkstoffen, Peptiden (mit einer Länge von 10 bis 100 Aminosäuren), Proteinen (mit einer Länge von mehr als 100 Aminosäuren), costimulatorischen Molekülen, Farbstoffen oder Kontrastmitteln.

Bevorzugte erfindungsgemäße anti-PSCA Antikörper sind mit Wirkstoffen, also mit pharmazeutisch wirksamen Stoffen, konjugiert. Bevorzugte Wirkstoffe umfassen Toxine, vorzugsweise Zytostatika, davon bevorzugt ausgewählt aus Maytansinoiden und Maytansinoid-Analoga, Taxoiden, CC-1065 und CC-1065 Analoga, Dolastatin und Dolastatin-Analoga, Methotrexat, Daunorubicin, Doxorubicin, Vincristin, Vinblastin, Melphalan, Mitomycin C, Chlorambucil und Calicheamicin.

Weiter bevorzugte erfindungsgemäße anti-PSCA Antikörper sind mit Kontrastmitteln konjugiert. Bevorzugte Kontrastmittel sind Radionuklide, davon bevorzugt die radioaktiven Isotope von Technetium, Rhenium, Yttrium, Kupfer, Gallium, Indium, Bismuth und Platin, insbesondere $^{99m}$Tc, $^{90}$Y, $^{186}$Re, $^{188}$Re, $^{68}$Ga und $^{111}$In.

Weiter bevorzugte erfindungsgemäße anti-PSCA Antikörper sind mit einem Protein, bevorzugt einem Enzym, vorzugsweise einem für das ADEPT-System geeigneten Enzym, einem costimulatorischen Molekül, vorzugsweise Toll-like Rezeptor Ligand, davon bevorzugt CpG oder mit einer Nukleinsäure konjugiert.

Weiter bevorzugte erfindungsgemäße anti-PSCA Antikörper sind mit einem Farbstoff, vorzugsweise einem Fluoreszenzfarbstoff konjugiert.

Weiter bevorzugte erfindungsgemäße Antikörper sind mit einem Peptid konjugiert, das eine Bindungsregion enthält, an die ein für das Peptid spezifischer Antikörper spezifisch bindet. Bevorzugt umfasst das Peptid eine 10 bis 50 Aminosäuren lange Peptidsequenz einer alpha-helikalen Region des humanen La-Proteins (die Aminosäuresequenz des humanen La Proteins entspricht SEQ ID No. 77). Besonders bevorzugt sind Peptide mit einer Aminosäure-Sequenzidentität von mindestens 90%, bevorzugt mindestens 95 %, besonders bevorzugt mindestens 99% zu einer der Aminosäuresequenzen gemäß SEQ ID No. 75 oder SEQ ID No. 76.

Besonders bevorzugt werden erfindungsgemäße anti-PSCA Antikörper rekombinant hergestellt. Vorzugsweise enthalten erfindungsgemäße rekombinante anti-PSCA Antikörper mindestens zwei unterschiedliche Bindungseinheiten, wobei

- mindestens eine der Bindungseinheiten spezifisch an PSCA bindet, wobei diese das Paratop des erfindungsgemäßen anti-PSCA Antikörpers enthält, und
- mindestens eine weitere der Bindungseinheiten spezifisch an ein anderes Antigen als PSCA, vorzugsweise an eine Oberflächenstruktur auf einer Effektorzelle bindet.

Die PSCA-bindende Bindungseinheit umfasst somit einen erfindungsgemäßen anti-PSCA Antikörper und enthält demzufolge zumindest die folgenden CDR-Regionen:

- CDR der variablen Region der leichten Kette umfassend die folgenden Aminosäure-Sequenzen: CDR1 SEQ ID No. 1, CDR2 SEQ ID No. 2, CDR3 SEQ ID No. 3 und

- CDR der variablen Region der schweren Kette umfassend die folgenden Aminosäure-Sequenzen: CDR1 SEQ ID No. 4, CDR2 SEQ ID No. 5, CDR3 SEQ ID No. 6

Unter einer "Bindungseinheit" im Sinne der Erfindung wird jegliche molekulare Struktur bezeichnet, die definierte Substanzen oder Strukturen spezifisch bindet. Abhängig von der Substanz oder Struktur, die gebunden wird, weist die Bindungseinheit dabei verschiedene Strukturen auf. Von der Bezeichnung "Bindungseinheit" im Sinne der Erfindung sind daher sowohl Antikörper umfasst (in diesem Fall enthält die Bindungseinheit zumindest das funktionale Paratop des Antikörpers) als auch andere Moleküle, vorzugsweise Proteine, die eine spezifische Bindung mit einem Antigen eingehen. Derartige Moleküle sind vorzugsweise Liganden, die spezifisch an eine Oberflächenstruktur (beispielsweise einen Oberflächenrezeptor) auf einer Zelle, insbesondere einer Effektorzelle, binden.

Bevorzugte Bindungseinheiten, die in erfindungsgemäßen Antikörpern enthalten sind, binden spezifisch an eine Effektorzelle. Die Definition von Effektorzellen im Sinne der Erfindung umfasst alle die Zellen des angeborenen und adaptiven Immunsystems, die Immunreaktionen vermitteln oder aktiv daran beteiligt sind. Bevorzugt ist die Effektorzelle ausgewählt aus T-Lymphozyten, NK-Zellen, Monozyten, Makrophagen, Dendritischen Zellen und Granulozyten. Besonders bevorzugt sind Bindungseinheiten, die gegen Oberflächenstrukturen auf T-Lymphozyten binden.

Enthält ein erfindungsgemäßer rekombinanter Antikörper mindestens zwei unterschiedliche Antikörper (mindestens einen erfindungsgemäßen anti-PSCA Antikörper und mindestens einen weiteren Antikörper, der nicht spezifisch an PSCA bindet), so liegt dieser bevorzugt in Form eines Diabody, Triabody oder Tetrabody vor. Bevorzugt davon sind bispezifische Antiköper aus Einzelketten-Antikörpern (single chain bispecific diabody, scBsDb) oder bispezifische Tandemantikörper aus Einzelketten-Antikörpern (single chain bispecific tandem antibody, scBsTaFv). Ganz besonders bevorzugt liegt ein erfindungsgemäßer rekombinanter Antikörper in Form eines scBsTaFv vor.

Bevorzugte nicht an PSCA-bindende Antikörper, die in einem erfindungsgemäßen rekombinanten Antikörper enthalten sind (in diesem Fall ist der rekombinante Antikörper ein bispezifischer Antikörper), sind Antikörper, die an eine Oberflächenstruktur einer Effektorzelle spezifisch binden. In diesem Fall enthält die nicht an PSCA-bindende Bindungseinheit zumindest das Paratop des Antikörpers, der an eine Oberflächenstruktur einer Effektorzelle bindet. Besonders bevorzugte sind die Antikörper gegen folgende Oberflächenstrukturen auf Effektorzellen gerichtet: CD3, CD8, CD4, CD25, CD28, CD16, NKG2D, NKp46, NKp44, aktivierende KIR-Rezeptoren (activating Killer Cell Immunoglobulin-like Receptors). Ganz besonders bevorzugt sind Antikörper, die gegen CD3 gerichtet sind (anti CD3-Antikörper), wobei der anti-CD3 Antikörper CDR Regionen mit den folgenden Aminosäuresequenzen enthält:

- CDR der variablen Region der schweren Kette: CDR1 SEQ ID No. 66, CDR2 SEQ ID No. 67, CDR3 SEQ ID No. 68 und

- CDR der variablen Region der leichten Kette: CDR1 SEQ ID No. 58, CDR2 SEQ ID No. 59, CDR3 SEQ ID No. 60.

Weiter bevorzugt sind rekombinante anti-PSCA Antikörper, die mindestens zwei unterschiedliche Bindungseinheiten enthalten, wobei mindestens eine weitere der Bindungseinheiten ein Ligand (bevorzugt ein Protein oder ein Glykan) ist, der an eine Oberflächenstruktur auf einer Effektorzelle spezifisch bindet. Bevorzugte Liganden beeinflussen die Aktivität der Effektorzellen durch deren Bindung an die (Effektor-)Zelloberfläche. Der Ligand ist dabei so ausgewählt, dass er spezifisch an Oberflächenstrukturen von Effektorzellen bindet und durch die Bindung Signalkaskaden zur Aktivierung der Effektorzellen auslöst. Bevorzugt ist als Ligand eine Proteinstruktur oder ein Glykan, welcher spezifisch an einen Rezeptor bindet, der spezifisch auf der Oberfläche von Effektorzellen exprimiert wird, wobei der Ligand durch seine Bindung an den Rezeptor eine Aktivierung der Effektorzelle bewirkt. Besonders bevorzugt sind die Proteinstrukturen ausgewählt aus ULB-Ps (z. B. ULB-P2), MICA, MICB und Zytokinen (wie z.B. IL2 und IL15).

Erfindungsgemäße Antikörper, insbesondere erfindungsgemäße rekombinante Antikörper, eignen sich zur spezifischen Bindung an PSCA-positive Zellen *in vivo* und *in vitro.* Daher umfasst die Erfindung auch den erfindungsgemäßen anti-PSCA Antikörper zur Verwendung als Arzneimittel, insbesondere zur Behandlung von Tumorerkrankungen, bevorzugt Prostatakrebs, oder als Diagnostikum.

Die Erfindung umfasst ferner die erfindungsgemäßen Antikörper zur Therapie von Tumorerkrankungen, insbesondere Prostatakrebs. Eine bevorzugte therapeutische Anwendung der erfindungsgemäßen (bevorzugt rekombinanten) Antikörper ist die Behandlung von Tumorerkrankungen, vorzugsweise Prostatakrebs. In der therapeutischen Anwendung werden die erfindungsgemäßen Antikörper bevorzugt zum Targeting von therapeutisch wirksamen Substanzen oder Effektorzellen zum Tumorgewebe eingesetzt. Zum Targeting von therapeutisch wirksamen Substanzen zum Tumorgewebe kommen bevorzugt erfindungsgemäße rekombinante Antikörper zum Einsatz, die mit einem Wirkstoff konjugiert sind. Zum Targeting von Effektorzellen zum Tumorgewebe werden bevorzugt erfindungsgemäße rekombinante Antikörper eingesetzt, die mindestens zwei unterschiedliche Bindungseinheiten enthalten, wobei mindestens eine der Bindungseinheiten ein erfindungsgemäßer anti-PSCA Antikörper ist und eine weitere der Bindungseinheiten spezifisch an eine Effektorzelle, vorzugsweise an CD3 auf T Zellen, bindet. Bevorzugt werden dafür die obenstehend genannten Antikörper eingesetzt.

Von der Erfindung ist auch eine pharmazeutische Zusammensetzung umfasst, die einen erfindungsgemäßen Antikörper in Assoziation mit einem pharmazeutisch annehmbaren Verdünnungsmittel oder Träger enthält. Bevorzugt liegt die erfindungsgemäße pharmazeutische Zusammensetzung in einer für die intravenöse Verabreichung geeigneten Form vor.

Vorzugsweise liegen die Antikörper in der erfindungsgemäßen pharmazeutischen Zusammensetzung in rekombinanter Form als chimäre oder besonders bevorzugt humanisierte Antikörper vor, die eine verringerte Immunogenität aufweisen.

Die erfindungsgemäßen pharmazeutischen Zusammensetzungen umfassen verschiedene Dosierungsformen und sind bevorzugt für die parenterale, besonders bevorzugt für die intravenöse Verabreichung geeignet. Vorzugsweise liegt die parenterale pharmazeutische Zusammensetzung in einer Darreichungsform vor, die zur Injektion geeignet ist. Besonders bevorzugte pharmazeutische Zusammensetzungen sind daher Lösungen, Emulsionen oder Suspensionen des Antikörpers in einem pharmazeutisch annehmbaren Verdünnungsmittel oder Träger.

Pharmazeutisch annehmbare Träger sind vorzugsweise sterile Flüssigkeiten, insbesondere Wasser, gepuffertes Wasser, 0,4% Salzlösung, 0,3% Glycin und dergleichen. Die pharmazeutischen Zusammensetzungen werden durch herkömmliche, gut bekannte Techniken sterilisiert. Die Zusammensetzungen enthalten bevorzugt pharmazeutisch akzeptable Hilfssubstanzen, wie etwa diejenigen, die erforderlich sind um näherungsweise physiologische Bedingungen zu ergeben und/oder die Stabilität der in der Zusammensetzung enthaltenen Antikörper zu erhöhen, wie etwa Mittel zur Einstellung des pH-Werts und Puffermittel, Mittel zur Einstellung der Toxizität und dergleichen, vorzugsweise ausgewählt aus Natriumacetat, Natriumchlorid, Kaliumchlorid, Calciumchlorid und Natriumlactat.

Bevorzugt ist die pharmazeutische Zusammensetzung eine injizierbare gepufferte Lösung, die zwischen 0,1 bis 500 mg/ml Antikörper, besonders bevorzugt zwischen 0,1 bis 250 mg/ml Antikörper, insbesondere zusammen mit 1 bis 500 mMol/l Puffer enthält. Die injizierbare Lösung liegt bevorzugt sowohl in einer flüssigen oder in einer lyophilisierten Dosierungsform vor. Der Puffer ist bevorzugt ausgewählt aus Histidin, Natriumsuccinat, Natriumcitrat, Natriumphosphat und Kaliumphosphat.

Bevorzugt enthält eine erfindungsgemäße pharmazeutische Zusammensetzung mindestens zwei unterschiedliche (bevorzugt rekombinante) Antikörper, wobei mindestens ein erfindungsgemäßer anti-PSCA Antikörper enthalten ist und mindestens ein weiterer rekombinanter Antikörper, der mit dem erfindungsgemäßen anti-PSCA Antikörper eine spezifische Bindung eingeht. Besonders bevorzugt sind die folgenden Kombinationen:

a) ein rekombinanter anti-PSCA Antikörper, der mit einem 10 bis 50 Aminosäuren langen Peptid konjugiert ist und ein weiterer rekombinanter Antikörper, der spezifisch an das Peptid bindet oder

b) ein bispezifischer anti-PSCA Antikörper, der zusätzlich einen Antikörper enthält, der an ein 10 bis 50 Aminosäuren langes Peptid bindet und ein weiterer rekombinanter Antikörper, der gegen ein anderes Antigen als PSCA bindet und der ein Peptid enthält, an das der bispezifische anti-PSCA Antikörper bindet.

Die mindestens zwei unterschiedlichen Antikörper liegen dabei vorzugsweise separat verpackt in der erfindungsgemäßen pharmazeutischen Zusammensetzung vor.

Eine bevorzugte pharmazeutische Zusammensetzung, die die unter a) definierte Kombination enthält, umfasst:

- Einen rekombinanten anti-PSCA Antikörper, der mit einem Peptid konjugiert ist, das eine 10 bis 50 Aminosäuren lange Aminosäuresequenz einer alpha-helikalen Region des humanen La-Proteins enthält (die Aminosäuresequenz des humanen La Proteins entspricht SEQ ID No. 77). Bevorzugt ist der anti-PSCA Antikörper mit einem Peptid mit einer Aminosäure-Sequenzidentität von mindestens 90%, bevorzugt mindestens 95 %, besonders bevorzugt mindestens 99% zu einer der Aminosäuresequenzen gemäß SEQ ID No. 75 oder SEQ ID No. 76 konjugiert.

- Einen weiteren (bevorzugt rekombinanten) Antikörper, der ein Paratop enthält, das spezifisch gegen das Peptid des anti-PSCA Antikörpers bindet und der eine weitere Bindungseinheit enthält, die spezifisch an eine Oberflächenstruktur einer Effektorzelle bindet. Davon bevorzugte Bindungseinheiten entsprechen den oben definierten. Für den Fall, dass der rekombinante anti-PSCA Antikörper mit einem Peptid mit einer Aminosäure-Sequenzidentität von mindestens 90%, bevorzugt mindestens 95 %, besonders bevorzugt mindestens 99% zu einer der Aminosäuresequenzen gemäß SEQ ID No. 75 oder SEQ ID No. 76 konjugiert ist, enthält der weitere rekombinante Antikörper vorzugsweise als Paratop, das an das Peptid bindet, die folgenden Aminosäuresequenzen:

  o CDR der variablen Region der leichten Kette: CDR1 SEQ ID No. 78, CDR2 SEQ ID No. 79, CDR3 SEQ ID No. 80 und CDR der variablen Region der schweren Kette: CDR1 SEQ ID No. 81, CDR2 SEQ ID No. 82, CDR3 SEQ ID No. 83 (hierin auch als "5B9"-Paratop bezeichnet) oder

  o CDR der variablen Region der leichten Kette: CDR1 SEQ ID No. 84, CDR2 SEQ ID No. 85, CDR3 SEQ ID No. 86 und CDR der variablen Region der schweren Kette: CDR1 SEQ ID No. 87, CDR2 SEQ ID No. 88, CDR3 SEQ ID No. 89 (hierin auch als "7B6"-Paratop bezeichnet).

Davon bevorzugte Kombinationen sind

- ein anti-PSCA Antikörper, der ein Peptid mit einer Aminosäure-Sequenzidentität von mindestens 90%, bevorzugt mindestens 95 %, besonders bevorzugt mindestens 99% zu einer der Aminosäuresequenzen gemäß SEQ ID No. 75 enthält mit einem weiteren Antikörper, der das 5B9 Paratop enthält oder

- ein anti-PSCA Antikörper, der ein Peptid mit einer Aminosäure-Sequenzidentität von mindestens 90%, bevorzugt mindestens 95 %, besonders bevorzugt mindestens 99% zu einer der Aminosäuresequenzen gemäß SEQ ID No. 76 enthält mit einem weiteren Antikörper, der das 7B6 Paratop enthält.

Eine bevorzugte pharmazeutische Zusammensetzung, die die unter b) definierte Kombination enthält, umfasst:

- Einen weiteren Antikörper, der spezifisch an eine Oberflächenstruktur einer Effektorzelle bindet und der eine 10 bis 50 Aminosäuren lange Aminosäuresequenz einer alpha-helikalen Region des humanen La-Proteins, vorzugsweise ein Peptid mit einer Aminosäure-Sequenzidentität von mindestens 90%, bevorzugt mindestens 95 %, besonders bevorzugt mindestens 99% zu einer der Aminosäuresequenzen gemäß SEQ ID No. 75 oder SEQ ID No. 76 enthält.

- Einen bispezifischen anti-PSCA Antikörper, der mit einem Antikörper konjugiert ist, der die 10 bis 50 Aminosäuren lange Aminosäuresequenz einer alpha-helikalen Region des humanen La-Proteins spezifisch bindet. Für den Fall, dass der weitere rekombinante Antikörper ein Peptid mit einer Aminosäure-Sequenzidentität von mindestens 90%, bevorzugt mindestens 95 %, besonders bevorzugt mindestens 99% zu einer der Aminosäuresequenzen gemäß SEQ ID No. 75 oder SEQ ID No. 76 enthält, enthält der bispezifische anti-PSCA Antikörper bevorzugt ein wie oben definiertes 5B9-Paratop oder 7B6-Paratop.

Davon bevorzugte Kombinationen sind

- ein bispezifischer anti-PSCA Antikörper der das 5B9 Paratop enthält und ein weiterer rekombinanter Antikörper, der ein Peptid mit einer Aminosäure-Sequenzidentität von mindestens 90%, bevorzugt mindestens 95 %, besonders bevorzugt mindestens 99% zu einer der Aminosäuresequenzen gemäß SEQ ID No. 75 enthält oder

- ein bispezifischer anti-PSCA Antikörper, der das 7B6 Paratop enthält und ein weiterer rekombinanter Antikörper, der ein Peptid mit einer Aminosäure-Sequenzidentität von mindestens 90%, bevorzugt mindestens 95 %, besonders bevorzugt mindestens 99% zu einer der Aminosäuresequenzen gemäß SEQ ID No. 76 enthält.

Eine bevorzugte diagnostische Anwendung ist die *in vivo* Diagnostik, wobei ein mit Kontrastmittel konjugierter erfindungsgemäßer anti-PSCA Antikörper zum gezielten Transport von Kontrastmitteln zum Tumorgewebe eingesetzt wird. Eine weiter bevorzugte diagnostische Anwendung der erfindungsgemäßen anti-PSCA Antikörper ist die *in vitro* Diagnostik, wobei bevorzugt ein mit einem Farbstoff konjugierter erfindungsgemäßer anti-PSCA Antikörper zum Nachweis PSCA-positiver Zellen in einer Probe, insbesondere in einer Gewebeprobe, eingesetzt wird.

Von der Erfindung ist auch eine diagnostische Zusammensetzung umfasst, die einen erfindungsgemäßen anti-PSCA Antikörper enthält. Der anti-PSCA Antikörper liegt darin vorzugsweise in einer Pufferlösung vor, vorzugsweise in gepufferter Salzlösung.

Die Erfindung umfasst ebenfalls eine Nukleinsäure, dessen Nukleotidsequenz für einen erfindungsgemäßen anti-PSCA Antikörper kodiert. Bevorzugt enthalten die für die CDR der variablen Regionen der leichten und schweren Kette kodierenden Abschnitte folgende Nukleotidsequenzen:

- CDR der variablen Region der leichten Kette: CDR1 SEQ ID No. 7, CDR2 SEQ ID No. 9, CDR3 SEQ ID No. 11 und CDR der variablen Region der schweren Kette: CDR1 SEQ ID No. 13, CDR2 SEQ ID No. 15, CDR3 SEQ ID No. 17 oder

- CDR der variablen Region der leichten Kette: CDR1 SEQ ID No. 8, CDR2 SEQ ID No. 10, CDR3 SEQ ID No. 12 und CDR der variablen Region der schweren Kette: CDR1 SEQ ID No. 14, CDR2 SEQ ID No. 16, CDR3 SEQ ID No. 18.

Der Begriff "Nukleinsäuren" im Sinne der Erfindung umfasst neben Desoxyribonukleinsäuren (DNA) und Ribonukleinsäuren (RNA) auch alle anderen linearen Polymeren in denen die Basen Adenin (A), Cytosin (C), Guanin (G) und Thymin (T) oder Uracil (U) in entsprechender Abfolge angeordnet sind (Nukleinsäuresequenz). Die Erfindung umfasst dabei auch die korrespondierenden RNA-Sequenzen (in denen Thymin durch Uracil ersetzt ist), komplementäre Sequenzen und Sequenzen mit modifiziertem Nukleinsäurerückgrat oder 3' oder 5'-Terminus. Der Begriff "Nukleinsäuresequenzen mit verändertem Rückgrat" umfasst dabei alle anderen linearen Polymere in denen die Basen Adenin (A), Cytosin (C), Guanin (G) und Thymin (T) oder Uracil (U) in entsprechender Abfolge angeordnet sind, wie z. B. Sequenzen mit einem Phosphothioat-, Phosphoramidat- oder O-Methyl-derivatisierten Rückgrat, Peptid-Nukleinsäuren (PNA) und locked nucleic acids (LNA) oder gemischtem Rückgrat. Der Begriff "modifizierter 3' oder 5' Terminus" umfasst dabei sowohl Modifikationen, die der Stabilisierung dienen als auch das Anbinden von Markern. Beispiele für Marker sind Enzyme, Farbstoffe oder Fluoreszenzfarbstoffe, Radionukleotide, sowie Haptene, wie z. B. Digoxigenin oder Biotin.

Von der Erfindung ist auch ein Vektor (auch: "Expressionsvektor") umfasst, der eine erfindungsgemäße Nukleinsäure enthält. Im Sinne der Erfindung wird unter einem Expressionsvektor ein Plasmid, Virus oder anderer Träger verstanden, der eine erfindungsgemäße Nukleinsäuresequenz rekombinant durch Insertion oder Inkorporation enthält. Der Expressionsvektor enthält typischerweise einen Replikationsstartpunkt, einen Promoter, sowie spezifische Gensequenzen, die eine phänotypische Selektion von den Expressionsvektor enthaltenden Wirtszellen ermöglichen.

Die Erfindung umfasst ferner eine Wirtszelle oder einen nicht-menschlichen Wirtsorganismus enthaltend eine erfindungsgemäße Nukleotidsequenz oder einen erfindungsgemäßen Vektor. Die Nukleotidsequenz oder der Vektor sind dabei rekombinant in der Wirtszelle oder dem nicht-menschlichen Wirtsorganismus enthalten.

Eine Wirtszelle im Sinne der Erfindung ist eine natürlich vorkommende Zelle oder eine transformiert oder genetisch veränderte Zelllinie, welche mindestens einen erfindungsgemäßen Vektor enthält. Die Erfindung umfasst dabei transiente Transfektanten (z. B. durch mRNA-Injektion) oder Wirtszellen, in denen mindestens ein erfindungsgemäßer Expressionsvektor als Plasmid oder künstliches Chromosom enthalten ist, sowie Wirtszellen in denen ein erfindungsgemäßer Expressionsvektor stabil in das Genom des Wirtes integriert ist.

Die Wirtszelle ist bevorzugt ausgewählt aus Zellen von Prokaryonten und Eukaryonten. Humane embryonale Stammzellen, die unter Vernichtung von Embryonen gewonnen wurden, sind keine Wirtszellen im Sinne der Erfindung. Bevorzugte Prokaryontenzellen sind ausgewählt aus Zellen von *Escherichia coli* und *Bacillus subtilis.* Bevorzugte Eukaryontenzellen sind ausgewählt aus Hefezellen (vorzugsweise *Saccharomyces cerevisiae* oder *Pichia pastoris*), Insektenzellen, amphibischen Zellen und Säugetierzellen (vorzugsweise CHO, HeLa, HEK293).

Nicht-menschliche Wirtsorganismen enthalten einen erfindungsgemäßen Vektor, der stabil in das Genom des Wirtsorganismus oder einzelner Zellen des Wirtsorganismus integriert ist. Bevorzugte Wirtsorganismen sind Pflanzen, Invertebraten oder Vertebraten, insbesondere *Bovidae, Drosophila melanogaster, Caenorhabditis elegans, Xenopus laevis,* Medaka, Zebrafisch oder *Mus musculus,* oder Zellen oder Embryonen der genannten Organismen.

Die Erfindung gibt anti-PSCA Antikörper und dazugehörige Expressionssysteme an, mit denen humanes PSCA effektiver gebunden werden kann. Dadurch eignet sich der erfindungsgemäße anti-PSCA Antikörper besonders für den Einsatz in Therapiesystemen, bei denen die Abtötung von Tumorzellen durch Rekrutierung von Effektorzellen vermittelt werden kann. Aufgrund der höheren Affinität des erfindungsgemäßen Antikörpers zu PSCA ist für eine Bindung (beispielsweise in der therapeutischen Anwendung) nur noch eine wesentlich geringere Menge an Antikörper erforderlich.

Es konnte gezeigt werden, dass der erfindungsgemäße anti-PSCA Antikörper in deutlich geringeren Konzentrationen und mit deutlich höherer Effizienz die spezifische Lyse von PSCA-positiven Tumorzellen vermitteln kann. Dies hat einerseits Kostenvorteile, da der Antikörperverbrauch reduziert werden kann. Andererseits in der therapeutischen Anwendung ist ein verbessertes Targeting vor allem auch von metastasierenden Zellen zu erwarten, sowie aufgrund der geringeren Einsatzmenge mit weniger Nebenwirkungen zu rechnen.

Anhand folgender Figuren und Ausführungsbeispiele soll die Erfindung näher erläutert werden, ohne die Erfindung auf diese zu beschränken.

Fig. 1 Schematische Darstellung unterschiedlicher erfindungsgemäßer rekombinanter Antikörper. A) Bispezifischer Antikörper in Form eines scBsTaFv enthaltend eine Bindungseinheit gegen PSCA und eine Bindungseinheit gegen CD3. B) Erfindungsgemäßer rekombinanter Antikörper gegen PSCA enthaltend ein Peptid-Tag (5B9), zur Verwendung mit dem ebenfalls dargestellten bispezifischen Antikörper in Form eines scBsTaFv enthaltend eine Bindungseinheit gegen CD3 und eine Bindungseinheit gegen die 5B9-Region des humanen La-Proteins. C) Erfindungsgemäßer rekombinanter Antikörper gegen PSCA enthaltend ein Peptid-Tag (7B6), zur Verwendung mit dem ebenfalls dargestellten bispezifischen Antikörper in Form eines scBsTaFv enthaltend eine Bindungseinheit gegen CD3 und eine Bindungseinheit gegen die 7B6-Region des humanen La-Proteins. Die jeweiligen $V_H$ und $V_L$ Untereinheiten sind über die Linkerpeptide mit den in den Abbildungen angegebenen Aminosäuresequenzen verknüpft.

Fig. 2 SDS-PAGE rekombinanter Antikörper. Spur 1: bispezifischer humanisierter Antikörper CD3-7B6 (scBsTaFv CD3-7B6); Spur 2: bispezifischer humanisierter Antikörper CD3-5B9 (scBsTaFv CD3($G_4$S)-5B9); Spur 3: erfindungsgemäßer bispezifischer muriner Antikörper CD3-PSCA(7F5) (scBsTaFv CD3-PSCA(7F5)); Spur 4: erfindungsgemäßer monospezifischer humanisierter Antikörper scFv PSCA(MB1)-7B6; Spur 5: bispezifischer humanisierter Antikörper CD3-PSCA(MB1) (scBsTaFv CD3-PSCA(MB1)); Spur 6: erfindungsgemäßer monospezifischer humanisierter Antikörper scFv PSCA(MB1)-5B9. M... Marker mit Fragmentgrößen (von oben nach unten): 70 kDa, 55 kDa, 40 kDa, 35 kDa, 25 kDa, 15 kDa.

Fig. 3 Spezifische Lyse $^{51}$Cr -beladener PC3-PSCA Tumorzellen im Versuch nach Ausführungsbeispiel 4 . Die Balken korrespondieren mit folgenden Versuchen: 1 (schwarz) ... humanisierter bispezifischer scBsTaFv CD3-PSCA(MB1) aus Ausführungsbeispiel 1; 2 (weiß) ... muriner bispezifischer scBsTaFv CD3-PSCA(MB1) aus Ausführungsbeispiel 1; 3 (gestreift) ... humanisierter bispezifischer scBsTaFv CD3-PSCA(7F5) (Vergleichsbeispiel); 4 (gepunktet) ... muriner bispezifischer scBsTaFv CD3-PSCA(7F5) (Vergleichsbeispiel).

Fig. 4 Ausgebildete Tumorfläche nach Transfer von PSCA-positiven Tumorzellen, T Zellen und Antikörper wie in Ausführungsbeispiel 5 beschrieben. A) Effektor-Target-Verhältnis 1:1; B) Effektor-Target-Verhältnis 10:1. Die nummerierten Linien korrespondieren mit folgenden Versuchen: 1 ... bispezifischer Kontrollantikörper anti-CD3xanti-5B9 (Kontrolle); 2 ... monospezifischer humanisierter scFv anti-PSCA(MB1) aus Ausführungsbeispiel 1 (Kontrolle); 3 ... Kontrollversuch ohne Antikörper (Negativkontrolle); 4 ... humanisierter bispezifischer scBsTaFv CD3-PSCA(MB1) aus Ausführungsbeispiel 1 (erfindungsgemäß); 5 ... humanisierter bispezifischer scBsTaFv CD3-PSCA(7F5) wie in Beispiel 3 beschrieben (Vergleichsbeispiel); 6 ... humanisierter bispezifischer scBsDb CD3xPSCA(7F5), der die PSCA-bindenden variablen Regionen des 7F5 Antikörpers enthält (Vergleichsbeispiel).

**Ausführungsbeispiel 1: Herstellung von bispezifischen rekombinanten Antikörpern (scBsTaFv), die spezifisch an PSCA binden (direktes Targeting)**

Zum Einsatz für das Targeting von PSCA$^+$ Zellen wurde ein bispezifischer Antikörper (single chain bispecific diabody, scBsTaFv) hergestellt, der mit einer Bindungseinheit an PSCA und mit der anderen Bindungseinheit an CD3 bindet. Der bispezifische Antikörper wird hierin auch vereinfacht als CD3-PSCA(MB1) bezeichnet und ist schematisch in Fig. 1A abgebildet.

Die PSCA-bindende Domäne, enthält die variable Region des erfindungsgemäßen anti-PSCA MB1 Antikörpers (muriner anti-PSCA Antikörper: schwere Kette SEQ ID No. 22, leichte Kette SEQ ID No. 20; humanisierter anti-PSCA Antikörper: schwere Kette SEQ ID No. 26, leichte Kette SEQ ID No. 24). Sie dient zur Bindung an die PSCA-positiven Tumorzellen. Die andere Domäne bindet an CD3, einen Bestandteil des T Zell Rezeptor Komplexes, und dient zur Aktivierung von T Zellen. Dadurch wird die Rekrutierung von T Zellen an die PSCA-positiven Zellen ermöglicht und vermittelt auf diese Weise die spezifische Lyse der PSCA-positiven Zellen durch die T Zellen.

Für die Generierung der monoklonalen anti-PSCA MB1 Antikörper wurden H-2d positive C3Hx Balb/c F1-Mäuse mit P815-Zellen immunisiert, die PSCA rekombinant auf der Oberfläche exprimierten. Durch die Fusion von Milzzellen und Myelomzellen entstanden Hybridomazellen, die monoklonale anti-PSCA Antikörper sezernierten. Nach Einzellklonierung dieser Hybridomazellen konnte der Klon MB1 selektioniert werden. Für die Generierung von rekombinanten anti-PSCA MB1 Antikörpern wurden die Nukleinsäuresequenzen der variablen Domäne der schweren ($V_H$) und leichten ($V_L$) Antikörperkette identifiziert. Dafür wurde zunächst die mRNA aus den anti-PSCA MB1 sezernierenden Hybridomazellen isoliert und in cDNA umgeschrieben. Anschließend wurde die variable Domäne der schweren Kette vom Isotyp IgG1 mit degenerierten Primern (Primerpaar SEQ ID No. 90 und 91) sowie die variable Domäne der leichten κ Kette mit Hilfe degenerierter Primer (Primerpaar SEQ ID No. 92 und 93) amplifiziert. Die PCR-Produkte wurden jeweils in den Vektor pGEM T-easy subkloniert und sequenziert.

Für die Klonierung des murinen Einzelkettenfragments (scFv) des anti-PSCA MB1 Antikörpers (hierin vereingacht als "scFv MB1" bezeichnet), bei dem die variable Region der schweren Kette über drei Glycin-Serin Linker ($G_4S$, SEQ ID No. 131) mit der variablen Region der leichten Kette verknüpft ist, wurde die Nukleinsäuresequenz der variablen Region der schweren Kette des anti-PSCA MB1 Antikörpers mit Hilfe des Primerpaares gemäß SEQ ID No. 94 und 95 amplifiziert. Die Nukleinsäuresequenz der variablen Region der leichten Kette des anti-PSCA MB1 Antikörpers wurde mit Hilfe des Primerpaares gemäß SEQ ID No. 96 und 97 amplifiziert. Die amplifizierten Nukleinsäuren wurden mittels overlap PCR zum scFv PSCA MB1 fusioniert und über SfiI und NotI in den eukaryotischen Expressionsvektor pSecTag2B kloniert (der Expressionsvektor wird hierin auch als "pSecTag2B_scFv MB1 murin" bezeichnet).

Für die Klonierung des bispezifischen Tandemantikörpers (scBsTaFv), der gegen PSCA und CD3 gerichtet ist und der die CDR Regionen des murinen MB1 Antikörpers enthält, wurde die Nukleinsäuresequenz eines murinen anti-CD3 scFv mit einem Primerpaar gemäß SEQ ID No. 98 und 99 amplifiziert und über ApaI 3'-seitig vom murinen scFv MB1 in den zuvor produzierten Expressionsvektor "pSecTag2B_scFv MB1 murin" kloniert, so dass der Vektor "pSecTag2B_ scBsTaFv PSCA(MB1)-CD3 murin" entstand.

Für die Klonierung des bispezifischen Tandemantikörpers (scBsTaFv), der gegen PSCA und CD3 gerichtet ist und der die CDR Regionen des humanisierten MB1 Antikörpers enthält, wurden die Gerüstregionen (FWR) der PSCA MB1 und CD3 Antikörper humanisiert. Dafür wurden die FWR der der variablen murinen Antikörperdomänen gegen die humanen FWR-Sequenzen eines hoch homologen humanen IgG1 ersetzt. Darüber hinaus wurden die humanisierten Antikörpersequenzen hinsichtlich ihrer Expression und Sekretion durch humane Zelllinien optimiert. Für die Humanisierung des murinen scFv CD3 bzw. scFv MB1 wurden zunächst die humanisierten $V_H$- und $V_L$-Sequenzen einzeln wie vorstehend beschrieben amplifiziert und anschließend über PCR zusammengefügt. Die Humanisierung der $V_H$- bzw. $V_L$-Sequenz erfolgte dabei durch die Zusammenlagerung von überlappenden Oligonukleotiden und anschließender Amplifikation der humanisierten $V_H$- bzw. $V_L$-Nukleinsäuresequenz. Um die Sequenzen der überlappenden Oligonukleotide festlegen zu können, wurde zunächst die murine $V_H$- bzw. $V_L$-Sequenz gegen eine humane IgG Datenbank (NCBI IgBlast) abgeglichen, der humane IgG mit der größten Homologie identifiziert und letztendlich die humanisierte $V_H$- bzw. $V_L$-Sequenz theoretisch erstellt und die überlappenden Oligonukleotide synthetisiert. Es wurden verschiedene bispezifische Antikörper konstruiert, bei denen unterschiedlich lange Linkerpeptide eingesetzt wurden.

Humanisierung des anti-CD3 Antikörpers

Für die Humanisierung der variablen Region der schweren Kette des anti-CD3 Antikörpers wurden Oligonukleotide gemäß SEQ ID No. 100 bis 105 zusammengelagert. Anschließend wurde die humanisierte Nukleinsäuresequenz der variablen Region der schweren Kette des anti-CD3 Antikörpers (mit einem $G_4S$-Linkerpeptid, SEQ ID No. 132) mit Hilfe des Primerpaares gemäß SEQ ID No. 105 und 106 amplifiziert. Für die Humanisierung der variablen Region der leichten Kette des anti-CD3 Antikörpers wurden Oligonukleotide gemäß SEQ ID No. 107 bis 112 zusammengelagert. Die humanisierte Nukleinsäuresequenz der variablen Region der leichten Kette des anti-CD3 Antikörpers wurde mit dem Primerpaar gemäß SEQ ID No. 112 und 113 amplifiziert.

Für die Generierung des humanisierten scFv des anti-CD3 Antikörpers, bei dem die variable Region der schweren Kette über einen Glycin-Serin Linker ($G_4S$) mit der variablen Region der leichten Kette verknüpft ist, wurde zuerst die humanisierte Nukleinsäuresequenz der variablen Region der schweren Kette des anti-CD3 Antikörpers über SfiI und BamHI in den Expressionsvektor pSecTag2B kloniert und anschließend über BamHI und NotI die humanisierte Nukleinsäuresequenz der variablen Region der leichten Kette des anti-CD3 Antikörpers stromabwärts davon in denselben Expressionsvektor gesetzt.

Für die Generierung des humanisierten scFv des anti-CD3 Antikörpers, bei dem die variable Region der schweren Kette über drei Glycin-Serin Linker ($G_4S$) mit der variablen Region der leichten Kette verknüpft ist, wurde zuerst die humanisierte Nukleinsäuresequenz der variablen Region der schweren Kette des anti-CD3 Antikörpers mit drei Glycin-Serin Linkern ($G_4S$) hergestellt. Dafür wurde die humanisierte Nukleinsäuresequenz der variablen Region der schweren Kette des anti-CD3 Antikörpers (mit drei $G_4S$-Linkerpeptiden) mit Hilfe des Primerpaares gemäß SEQ ID No. 105 und 114 amplifiziert und über SfiI und BamHI in den Expressionsvektor pSecTag2B kloniert und anschließend über BamHI und NotI die humanisierte Nukleinsäuresequenz der variablen Region der leichten Kette des anti-CD3 Antikörpers stromabwärts davon in denselben Expressionsvektor gesetzt.

Humanisierung des anti-PSCA MB1 Antikörpers

Für die Humanisierung der variablen Region der leichten Kette des anti-PSCA MB1 Antikörpers wurden Oligonukleotide gemäß SEQ ID No. 115 bis 118 zusammengelagert. Die humanisierte Nukleinsäuresequenz der variablen Region der leichten Kette des anti-PSCA MB1 Antikörpers wurde mit dem Primerpaar gemäß SEQ ID No. 119 und 120 amplifiziert.

Für die Humanisierung der variablen Region der schweren Kette des anti-PSCA MB1 Antikörpers wurden Oligonukleotide gemäß SEQ ID No. 121 bis 125 zusammengelagert. Die humanisierte Nukleinsäuresequenz der variablen Region der schweren Kette des anti-PSCA MB1 Antikörpers wurde mit dem Primerpaar gemäß SEQ ID No. 126 und 127 amplifiziert.

Herstellung des bispezifischen humanisierten Antikörpers scBsTaFv CD3-PSCA(MB1)

Schließlich wurden die humanisierten Nukleinsäuresequenzen der variablen Region der schweren Kette und der variablen Region der leichten Kette des MB1 Antikörpers mittels overlap PCR unter Verwendung der Primer gemäß SEQ ID No. 128 und129 zusammengelagert und amplifiziert. Die so generierte Nukleinsäuresequenz codierend für den humanisierten scFv MB1 (in der Organisation $V_L$-$G_4SG_4SGASAAG_4SG_4S$-$V_H$, Linkerpeptid gemäß SEQ ID No. 130) wurde über XhoI und ApaI stromabwärts von dem oben beschriebenen humanisierten scFv des anti-CD3 Antikörpers (mit drei $G_4S$-Linkerpeptiden) kloniert, wobei der Expressionsvektor "pSecTag2B_ scBsTaFv PSCA(MB1)-CD3 human" entstand.

Zur Expression des bispezifischen humanisierten Antikörpers gemäß Fig. 1A wurden Hek293T Zellen mit dem Expressionsvektor transfiziert und die sezernierten Antikörper aus den Zellkulturüberständen mittels Nickel-Affinitätschromatographie gegebenenfalls in Kombination mit einer fraktionierten Ammoniumsulfatfällung aufgereinigt. Fig. 2 Spur 5 zeigt eine SDS-Gelelektrophorese Aufnahme des aufgereinigten bispezifischen Antikörpers.

**Ausführungsbeispiel 2: Herstellung von bispezifischen rekombinanten Antikörpern, die spezifisch an PSCA binden (modulares Targeting)**

Zur Bereitstellung einer erfindungsgemäßen pharmazeutischen Zusammensetzung ("modulares Targeting System 1", schematisch in Fig. 1B dargestellt) wurden folgende rekombinante Antikörper hergestellt:

- erfindungsgemäßer rekombinanter anti-PSCA Antikörper enthaltend ein Peptid gemäß SEQ ID No. 75 (hierin auch "E5B9"). Dieser Antikörper wird im Folgenden als scFv PSCA(MB1)-E5B9 bezeichnet.

- bispezifischer Antikörper (scBsTaFv), der gegen CD3 und das Peptid gemäß SEQ ID No. 75 gerichtet ist. Das gegen CD3 gerichtete Paratop umfasst die folgenden Aminosäuresequenzen der variablen Regionen: schwere Kette SEQ ID No. 65, leichte Kette SEQ ID No. 57. Das gegen das Peptid gemäß SEQ ID No. 75 gerichtete Paratop umfasst folgende Aminosäuresequenzen der hypervariablen Regionen der variablen Regionen: leichte Kette CDR1 SEQ ID No. 78, CDR2 SEQ ID No. 79, CDR3 SEQ ID No. 80, schwere Kette CDR1 SEQ ID No. 81, CDR2 SEQ ID No. 82, CDR3 SEQ ID No. 83. Dieser Antikörper wird im Folgenden als scBsTaFv CD3-5B9 bezeichnet. Es wurden zwei unterschiedliche scBsTaFv CD3-5B9 hergestellt, die sich lediglich voneinander in dem Linkerpeptid zwischen VH und VL des anti-CD3Antikörpers unterscheiden (siehe Fig. 1B).

Klonierung des humanisierten scFv PSCA(MB1)-E5B9:

Für die Generierung des humanisierten scFv PSCA(MB1) mit E5B9-Epitop am C-Terminus (MB1 [$V_L$-$G_4SG_4SGASAAG_4SG_4S$-MB1 $V_H$]-[$G_4S$-E5B9], dargestellt in Fig. 1B unten) wurde der in Ausführungsbeispiel 1 beschriebene humanisierte scFv PSCA(MB1) (MB1 $V_L$-$G_4SG_4SGASAAG_4SG_4S$-MB1 $V_H$-$G_4S$, Linkerpeptide gemäß SEQ ID No. 130 und 132) mittels der Primer gemäß SEQ ID No. 134 und 135 amplifiziert und anschließend über SfiI und NotI in pSecTag2B kloniert. Nach Zusammenlagerung der Oligonukleotide gemäß SEQ ID No. 136 und 137 wurde die Nukleinsäuresequenz für E5B9 über NotI und XhoI 3'-seitig vom humanisierten scFv PSCA(MB1) einkloniert, wobei das Konstrukt "pSecTag2B_ scFv PSCA(MB1)-E5B9 humanisiert" entstand.

Klonierung zweier an T-Zellen und an das E5B9-Peptid bindender Effektormodule "scBsTaFv CD3($G_4S$)-5B9" und "scBsTaFv CD3(3x$G_4S$)-5B9"

Wie schematisch in Fig. 1B dargestellt ist, wurden zum Aufbau des modularen Targetingsystems I umfassend das scFv PSCA(MB1)-5B9 zwei sogenannte Effektormodule (bispezifische Antikörper scBsTaFv CD3-5B9) hergestellt, die sich in der Anzahl der Glycin-Serin ($G_4S$) Elemente des Linkerpeptids zwischen der $V_H$ und $V_L$-Kette der anti-CD3 Domäne unterscheiden und deshalb als "scBsTaFv CD3($G_4S$)-5B9" bzw. "scBsTaFv CD3(3x$G_4S$)-5B9" bezeichnet wurden.

- Klonierung des "scBsTaFv CD3($G_4S$)-5B9" humanisiert (Schema siehe Fig. 1B oben):

  Für die Humanisierung der variablen Region der schweren Kette des 5B9 Antikörpers (spezifisch gegen das Peptid E5B9 gerichtet) wurden Oligonukleotide gemäß SEQ ID No. 138 bis 142 zusammengelagert. Anschließend wurde die humanisierte Nukleinsäuresequenz der variablen Region der schweren Kette des 5B9 Antikörpers (mit einem $G_4S$-Linkerpeptid, SEQ ID No. 132) mit Hilfe des Primerpaares gemäß SEQ ID No. 143 und 144 amplifiziert. Für die Humanisierung der variablen Region der leichten Kette des 5B9 Antikörpers wurden Oligonukleotide gemäß SEQ ID No. 145 bis 149 zusammengelagert. Die humanisierte Nukleinsäuresequenz der variablen Region der leichten Kette des 5B9 Antikörpers wurde mit dem Primerpaar gemäß SEQ ID No. 150 und 151 amplifiziert.

  Schließlich wurden die humanisierten Nukleinsäuresequenzen für 5B9 $V_H$ und 5B9 $V_L$ mittels overlap PCR unter Verwendung der Primer gemäß SEQ ID No. 152 und 153 zusammengelagert und amplifiziert. Die so generierte Nukleinsäuresequenz für den humanisierten scFv 5B9 ($V_H$-3x$G_4S$-$V_L$) wurde über XhoI und ApaI stromabwärts vom humanisierten scFv CD3 $V_H$-$G_4S$-$V_L$ in den "pSecTag2B_scFv CD3 $V_H$-$G_4S$-$V_L$" humanisiert kloniert, wobei der Vektor "pSecTag2B_scBsTaFv CD3($G_4S$)-5B9 humanisiert" entstand.

- Klonierung des "scBsTaFv CD3(3x$G_4S$)-5B9" humanisiert (Schema siehe Fig. 1B Mitte):
  Die humanisierte Sequenz für scFv 5B9 ($V_H$-3x$G_4S$-$V_L$) wurde über XhoI und ApaI stromabwärts vom humanisierten scFv CD3 ($V_H$-3x$G_4S$-$V_L$) in den "pSecTag2B_scFv CD3 $V_H$-3x$G_4S$-$V_L$" humanisiert kloniert, wobei der Vektor "pSecTag2B_scBsTaFv CD3(3x$G_4S$)-5B9 humanisiert" entstand.

Zur Bereitstellung einer erfindungsgemäßen pharmazeutischen Zusammensetzung ("modulares Targeting System 2", schematisch in Fig. 1C dargestellt) wurden folgende rekombinante Antikörper hergestellt:

- erfindungsgemäßer rekombinanter anti-PSCA Antikörper enthaltend ein Peptid gemäß SEQ ID No. 76 (hierin auch als "E7B6" bezeichnet). Dieser Antikörper wird im Folgenden als scFv PSCA(MB1)-E7B6 bezeichnet.

- bispezifischer Antikörper (scBsTaFv), der gegen CD3 und das Peptid gemäß SEQ ID No. 76 gerichtet ist. Das gegen CD3 gerichtete Paratop umfasst die folgenden Aminosäuresequenzen der variablen Regionen: schwere Kette SEQ ID No. 65, leichte Kette SEQ ID No. 57. Das gegen das Peptid gemäß SEQ ID No. 76 gerichtete Paratop umfasst folgende Aminosäuresequenzen der hypervariablen Regionen der variablen Regionen: leichte Kette CDR1 SEQ ID No. 84, CDR2 SEQ ID No. 85, CDR3 SEQ ID No. 86, schwere Kette CDR1 SEQ ID No. 87, CDR2 SEQ ID No. 88, CDR3 SEQ ID No. 89. Dieser Antikörper wird im Folgenden als scBsTaFv CD3-7B6 bezeichnet.

Klonierung des humanisierten scFv PSCA(MB1)-E7B6:

Für die Generierung des humanisierten scFv PSCA(MB1) mit E7B6-Epitop am C-Terminus MB1 [VL-$G_4SG_4SGASAAG_4SG_4S$-MB1 $V_H$]-[$G_4S$-E7B6], dargestellt in Fig. 1C unten) wurde zunächst die Zusammenlagerung der Oligonukleotide gemäß SEQ ID No. 154 und 155 durchgeführt und anschließend die Klonierung der E7B6-Nukleinsäuresequenz über NotI und XhoI 3'-seitig in "pSecTag2B_ scFv PSCA(MB1) humanisiert" ($V_L$-$G_4SG_4SGASAAG_4SG_4S$-$V_H$-$G_4S$, analog zum oben beschriebenen Konstrukt mit dem E5B9 Peptid), wobei der Vektor "pSecTag2B_ scFv PSCA(MB1)-E7B6 humanisiert" entstand.

Klonierung eines an T-Zellen und an das E7B6-Peptid bindenden Effektormoduls "scBsTaFv CD3(3x$G_4S$)-7B6" humanisiert:

Für die Humanisierung der variablen Region der schweren Kette des 7B6 Antikörpers (spezifisch gegen das Peptid E7B6 gerichtet) wurden überlappende Oligonukleotide gemäß SEQ ID No. 156 bis 160 zusammengelagert. Anschließend wurde die humanisierte Nukleinsäuresequenz der variablen Region der schweren Kette des 7B6 Antikörpers (mit einem $G_4S$-Linkerpeptid, SEQ ID No. 132) mit Hilfe des Primerpaares gemäß SEQ ID No. 161 und 162 amplifiziert. Für die Humanisierung der variablen Region der leichten Kette des 7B6 Antikörpers wurden Oligonukleotide gemäß SEQ ID No. 163 bis 167 zusammengelagert. Die humanisierte Nukleinsäuresequenz der variablen Region der leichten Kette des 7B6 Antikörpers wurde mit dem Primerpaar gemäß SEQ ID No. 168 und 169 amplifiziert.

Schließlich wurden die humanisierten Nukleinsäuresequenzen für 7B6 $V_H$ und 7B6 $V_L$ mittels overlap PCR unter Verwendung der Primer gemäß SEQ ID No. 170 und 171 zusammengelagert und amplifiziert. Die so generierte Nukleinsäuresequenz für den humanisierten scFv 7B6 ($V_H$-3x$G_4$S-$V_L$) wurde über XhoI und ApaI stromabwärts vom humanisierten scFv CD3 $V_H$-$G_4$S-$V_L$ in den "pSecTag2B_scFv CD3 $V_H$-$G_4$S-$V_L$" humanisiert kloniert, wobei der Vektor "pSecTag2B_scBsTaFv CD3($G_4$S)-7B6 humanisiert" entstand.

Zur Expression der einzelnen Fusionsproteine der modularen Targeting Systeme 1 und 2 wurden Hek293T Zellen mit den Expressionsvektoren transfiziert und die sezernierten rekombinanten scFv (scFv PSCA-E5B9 oder scFv PSCA-E7B6) und die bispezifischen Antikörper (scBsTaFv CD3($G_4$S)-5B9 bzw. scBsTaFv CD3(3x$G_4$S)-5B9 und scBsTaFv CD3-7B6) aus dem Zellkulturüberstand mit Hilfe von Affinitätschromatographie über Ni-NTA Agarose (Qiagen, Hilden, Deutschland) (gegebenenfalls in Kombination mittels einer fraktionierten Ammoniumsulfatfällung) aufgereinigt. Per SDS-Page und Immunoblot wurde die Reinheit und Stabilität der rekombinanten Antikörperderivate nachgewiesen (Fig. 2)

**Ausführungsbeispiel 3: Bestimmung der Dissoziationskonstante der Bindung an PSCA**

Die Ermittlung der Affinitätskonstante für die jeweilige anti-PSCA Domäne der rekombinanten, bispezifischen Antikörper mit dem anti-PSCA MB1 Antikörper (erfindungsgemäß) und dem anti-PSCA 7F5 Antikörper (Vergleichsbeispiel, Antikörper aus [Feldmann 2010]) basierte auf einer durchflusszytometrischen Analyse der Bindung an PSCA-positive PC3 Zellen.

Für die Generierung der Bindungskurven der rekombinanten anti-PSCA Antikörperdomänen wurden jeweils $2x10^5$ PSCA-positive Zellen mit 100 μl der bispezifischen Antikörper (MB1 und 7F5) für 1 h bei 4°C inkubiert. Die Antikörper wurden jeweils in folgenden Konzentrationen eingesetzt:

| Antikörpermenge je Ansatz in ng | Antikörper-Konzentration in pmol/l |
|---|---|
| 1000 | 90000 |
| 100 | 9000 |
| 50 | 4500 |
| 10 | 900 |
| 5 | 450 |
| 1 | 90 |
| 0,5 | 45 |
| 0,1 | 9 |
| 0,05 | 4,5 |
| 0,01 | 0,9 |
| 0,001 | 0,09 |

Um die spezifische Bindung der rekombinanten CD3-PSCA Antikörper nachzuweisen, wurde ein Maus-anti-c-myc IgG-FITC Nachweisantikörper (AbD Serotec, Düsseldorf, Deutschland) verwendet, der nach Abschluss des ersten Färbeschrittes für 30 min bei 4°C mit den anti-PSCA Antikörpermarkierten PSCA-positiven Zellen inkubiert wurde. Als Negativkontrolle wurde eine Probe mitgeführt, in der PSCA-positive Zellen nur mit dem Nachweisantikörper Maus-anti-c-myc IgG-FITC angefärbt wurde. Die Zellen wurden an einem BD FACS Calibur Flow Cytometer (BD Biosciences Pharmingen, Heidelberg, Deutschland) analysiert und die Daten wurden mit Hilfe der Software WinMDI 2.8 (Joseph Trotter, La Jolla, CA USA) ausgewertet.

Zur Auswertung wurden die ermittelten MFI-Werte (mittlere Fluoreszenzintensitätswerte) gegen die getesteten Antikörperkonzentrationen aufgetragen und jeweils eine Trendlinie vom polynomischen Regressionstyp zweiter Ordnung berechnet. Um die Affinitätskonstante zu ermitteln, wurde eine Trendlinie vom polynomischen Regressionstyp zweiter Ordnung berechnet und eingefügt. Die daraus resultierende Bindungskurve diente als Grundlage zur Berechnung der Affinitätskonstanten $K_D$, welche als diejenige Konzentration definiert ist, die beim 50%-Wert des maximalen MFI-Wertes und somit bei halbmaximaler Sättigung der Bindung erreicht wird. Um diese Konstante zu berechnen, wurde zunächst die erste Ableitung der Bindungskurve, welche einer quadratischen Funktion folgt, gebildet. Um ausgehend von dieser Gleichung das Maximum der Funktion zu ermitteln, wurde die erste Ableitung "Null" gesetzt und nach x aufgelöst. Durch Einsetzen des erhaltenen x-Wertes in die Ausgangsgleichung wurde das Maximum der Funktion $y_{max}$ und somit $y_{max}/2$, welches dem MFI-Wert bei halbmaximaler Absättigung der Bindungsstellen entspricht, errechnet. Da der $K_D$-Wert dem x-Wert bei $y_{max}/2$ entspricht, kann dieser durch Einsetzen des $y_{max}/2$-Wertes in die quadratische Funktion der Bindungskurve und Umstellen der Gleichung nach x abschließend kalkuliert werden

Folgende $K_D$-Werte wurden auf diese Weise für die Bindung an PSCA ermittelt:

| | $K_D$ |
|---|---|
| scBsTaFv CD3xPSCA(MB1) | $6,3 \times 10^{-7}$ |
| scBsTaFv CD3xPSCA(7F5) | $2,3 \times 10^{-6}$ |

Es konnte gezeigt damit werden, dass die Affinität des erfindungsgemäßen anti-PSCA Antikörper zum Antigen PSCA eine Größenordnung höher ist, als die des aus dem Stand der Technik bekannten Antikörpers 7F5.

**Ausführungsbeispiel 4: Spezifische Lyse von PSCA+ Zellen mit bispezifischen anti-PSCA Antikörpern**

Im Chromfreisetzungstest wurden voraktivierte PBMCs $5x10^4$ mit $5x10^3$ $^{51}$Cr-beladene PC3-PSCA Tumorzellen (Effektor-Target-Verhältnis = 10:1) in An- und Abwesenheit der rekombinanten Antikörper in RPMI Medium in einem Gesamtvolumen von 200μl kokultiviert. Dazu wurden die erfindungsgemäßen Antikörper aus Beispiel 1 eingesetzt (sowohl in muriner als auch in humanisierter Form). Als Vergleichsbeispiel wurde der aus dem Stand der Technik bekannter muriner anti-PSCA(7F5) Antikörper in einem gleichartig aufgebauten bispezifischen Antikörper eingesetzt. Die variablen Regionen des 7F5 Antikörpers entsprechen: schwere Kette SEQ ID No. 48, leichte Kette SEQ ID No. 50. Es wurden Versuche mit folgenden konkreten Antikörperkonstrukten durchgeführt:

1. humanisierter bispezifischer scBsTaFv CD3-PSCA(MB1) aus Ausführungsbeispiel 1
2. muriner bispezifischer scBsTaFv CD3-PSCA(MB1) aus Ausführungsbeispiel 1
3. humanisierter bispezifischer scBsTaFv CD3-PSCA(7F5) (Vergleichsbeispiel)
4. muriner bispezifischer scBsTaFv CD3-PSCA(7F5) (Vergleichsbeispiel)

Nach 20h Inkubation bei 37°C im Brutschrank wurde das ins Medium freigesetzte Chrom gemessen. Die spezifische Lyse wurde wie folgt berechnet:

Spezifische Lyse in % = [freigesetztes $^{51}$Cr - spontan freigesetztes $^{51}$Cr (Minimum)]/[maximal freigesetztes $^{51}$Cr (Maximum) - Minimum] x 100%

Die Ergebnisse des *in vitro* Versuchs sind graphisch in Fig. 3 dargestellt. Dargestellt ist der prozentuale Anteil lysierter PC3 Zellen an den insgesamt eingesetzten PC3 Zellen (y-Achse). Während die erfindungsgemäßen Antikörper der Versuchsgruppen 1 und 2 bereits bei Mengen von weniger als 1 ng eine starke Lyse der PSCA-positiven Zellen zeigen, ist bei dem bekannten 7F5 Antikörper in einem vergleichbaren Konstrukt (Versuchsgruppen 3 und 4) eine detektierbare Lyse erst beim Einsatz mehrerer ng des Antikörpers festzustellen. Maximal wurde eine Lyse von etwa 60% der eingesetzten PC3 Zellen festgestellt. Mit den erfindungsgemäßen Antikörpern konnten bereits beim Einsatz von einer deutlich geringeren Antikörpermenge eine Lyse von etwa 80 % der eingesetzten PC3 Zellen festgestellt werden. Maximal konnten mehr als 90 % der eingesetzten PC3 Zellen mit einem erfindungsgemäßen Antikörper (Versuchsgruppe 1) lysiert werden.

**Ausführungsbeispiel 5: Hemmung des Tumorwachstums durch scBsTaFv CD3-PSCA(MB1) humanisiert *in vivo***

Zum Nachweis der Wirksamkeit des erfindungsgemäßen anti-PSCA MB1 Antikörpers wurde der Effekt eines davon abgeleiteten bispezifischen Antikörpers scBsTaFv CD3-PSCA(MB1), der nach Ausführungsbeispiel 1 hergestellt wurde (schematisch dargestellt in Fig. 1A, oben), im murinen Tumormodell getestet. Als Modellorganismus wurden Nacktmäuse eingesetzt, die nach Transfer von PSCA-positiven PC3 Tumorzellen Tumoren ausbilden. Im Versuch wurden PSCA-positiven Tumorzellen in Kombination mit T Zellen und einem Antikörper transferiert.
Folgende Antikörper wurden dafür eingesetzt, wobei je Maus 10 μg Antikörper injiziert wurden:

(1) bispezifischer Kontrollantikörper anti-CD3xanti-5B9 (Kontrolle)

(2) monospezifischer humanisierter scFv anti-PSCA(MBl) aus Ausführungsbeispiel 1 (Kontrolle)

(3) Kontrollversuch ohne Antikörper (Negativkontrolle)

(4) humanisierter bispezifischer scBsTaFv CD3-PSCA(MB1) aus Ausführungsbeispiel 1

(5) humanisierter bispezifischer scBsTaFv CD3-PSCA(7F5) wie in Beispiel 3 beschrieben (Vergleichsbeispiel)

(6) humanisierter bispezifischer scBsDb CD3xPSCA(7F5), der die PSCA-bindenden variablen Regionen des 7F5 Antikörpers enthält (Vergleichsbeispiel)

Die Wirkung der bispezifischen Antikörper beruht auf der Rekrutierung der CD3-positiven T-Zellen zu den PSCA-positiven Tumorzellen und der dadurch vermittelten Lyse der Tumorzellen.

In einem ersten Versuch wurden $5x10^3$ PSCA-positive Tumorzellen in jeweils 8 Nacktmäuse pro Versuchsgruppe und $5x10^3$ T-Zellen transferiert (Effektor-Target-Verhältnis 1:1). In den Kontrollgruppen (1) und (3) wurde in Abwesenheit eines anti-PSCA Antikörpers rapides Tumorwachstum festgestellt. Auch der Kontrollversuch (2), bei dem der monospezifische scFv anti-PSCA(MB1) injiziert wurde, zeigte rapides Tumorwachstum. Dadurch lässt sich ein cytotoxischer Effekt des monospezifischen Antikörpers ausschließen und die Wirksamkeit allein dem bispezifischen Konstrukt zugeschrieben werden. Die Tumorfläche der gebildeten Tumoren wurde 25 Tage nach Zelltransfer ermittelt. Die drei Kontrollgruppen zeigten keinen signifikanten Unterschied der Tumorfläche (Fig. 4A).

Bei Transfer der bispezifischen Vergleichsantikörpers, die die variablen Regionen des anti-PSCA 7F5 Antikörpers enthalten (Vergleichsgruppen (5) in Form eines Tandemantikörpers und (6) in Form eines Diabodys) wurde ebenfalls ein rapides Tumorwachstum festgestellt (Daten nicht dargestellt). Auch in einem zweiten Versuch, bei der die Tumorzellen in einem Effektor-Target-Verhältnis von 10:1 (also bei 10-facher Menge T Zellen; Transfer von $5x10^3$ PSCA-positiven Tumorzellen und $5x10^4$ T Zellen) wurde ein rapides Tumorwachstum festgestellt, dass keinen statistisch signifikanten Unterschied zur Kontrollgruppe (1) zeigte (Fig. 4B). Das Tumorwachstum wurde durch die anti-PSCA 7F5 Antikörper nicht gehemmt, sondern lediglich leicht verzögert. Auch bei T Zell Überschuss kann somit keine *in vivo* Hemmung des Tumorwachstums durch den anti-PSCA 7F5 Antikörper festgestellt werden.

Der Transfer des erfindungsgemäßen bispezifischen Antikörpers scBsTaFv CD3-PSCA(MB1) aus Ausführungsbeispiel 1 bei einem Effektor-Target-Verhältnis 1:1 konnte das Tumorwachstum signifikant verringern. Während 25 Tage nach Zelltransfer in 2 der 8 Versuchstiere Tumoren mit einer deutlich geringen Fläche (Durchmesser etwa 2 mm) auftraten, blieben 6 der 8 Versuchstiere tumorfrei (Fig 4A). Der erfindungsgemäße anti-PSCA MB1 Antikörper (Versuchsgruppe (4)) ist somit dem vergleichbar aufgebautem anti-PSCA 7F5 Antikörper (Versuchsgruppe (5)) hinsichtlich der *in vivo* Wirksamkeit unter identischen Versuchsbedingungen deutlich überlegen.

**Zitierte Nichtpatentliteratur**

Feldmann A, Stamova S, Bippes CC, Bartsch H, Wehner R, Schmitz M, Temme A, Cartellieri M, Bachmann M. Retargeting of T cells to prostate stem cell antigen expressing tumor cells: comparison of different antibody formats. Prostate. 2011 Jun 15;71(9):998-1011. doi: 10.1002/pros.21315. Epub 2010 Dec 28.

Gu Z, Yamashiro J, Kono E, Reiter RE. Anti-prostate stem cell antigen monoclonal antibody 1G8 induces cell death in vitro and inhibits tumor growth in vivo via a Fc-independent mechanism. Cancer Res. 2005 Oct 15;65(20):9495-500.

Morgenroth A, Cartellieri M, Schmitz M, Günes S, Weigle B, Bachmann M, Abken H, Rieber EP, Temme A. Targeting of tumor cells expressing the prostate stem cell antigen (PSCA) using genetically engineered T-cells. Prostate. 2007 Jul 1;67(10):1121-31.

Reiter, R.E., Gu, Z., Watabe, T., Thomas, G., Szigeti, K., Davis, E., Wahl, M., Nisitani, S., Yamashiro, J., Le Beau, M.M., Loda, M. und Witte, O.N. Prostate stem cell antigen: A cell surface marker overexpressed in prostate cancer. Proc Natl Acad Sci USA. 95(4): 1735-40 (1998)

Roehl, K.A., M. Han, C. G. Ramos, J. A. V. Antenor, and W. J. Catalona. Cancer progression and survival rates following anatomical radical retropubic prostatectomy in 3,478 consecutive patients: long-term results. J Urol, 172(3):910-4, Sep 2004.

Thomas-Kaskel, A.-K., R. Zeiser, R. Jochim, C. Robbel, W. Schultze-Seemann, C. F. Waller, and H. Veelken. Vaccination of advanced prostate cancer patients with PSCA and PSA peptide-loaded dendritic cells induces DTH responses that correlate with superior overall survival. Int J Cancer, 119(10):2428-34, Nov 2006.

**Patentansprüche**

1. An Prostata-spezifisches Stammzellantigen bindender Antikörper, wobei

   - die komplementaritätsbestimmenden Regionen (CDR) der variablen Region der leichten Kette die folgenden Aminosäure-Sequenzen umfassen: CDR1 SEQ ID No. 1, CDR2 SEQ ID No. 2, CDR3 SEQ ID No. 3 und
   - die CDR der variablen Region der schweren Kette die folgenden Aminosäure-Sequenzen umfassen: CDR1 SEQ ID No. 4, CDR2 SEQ ID No. 5, CDR3 SEQ ID No. 6.

2. Antikörper nach Anspruch 1, dessen variable Regionen eine humanisierte Aminosäuresequenz aufweisen.

3. Antikörper nach Anspruch 1 oder 2, umfassend mindestens zwei unterschiedliche Bindungseinheiten, wobei

- mindestens eine der Bindungseinheiten an Prostata-spezifisches Stammzellantigen bindet und die in Anspruch 1 definierten komplementaritätsbestimmenden Regionen umfasst und
- mindestens eine weitere der Bindungseinheiten spezifisch an ein anderes Antigen als Prostata-spezifisches Stammzellantigen bindet.

4. Antikörper nach Anspruch 3, wobei die weitere Bindungseinheit spezifisch an eine Oberflächenstruktur auf einer Effektorzelle bindet, wobei die Bindungseinheit vorzugsweise ausgewählt ist aus einer Bindungseinheit eines an eine Oberflächenstruktur einer Effektorzelle bindenden Antikörpers oder Liganden.

5. Antikörper nach Anspruch 4, wobei die Effektorzelle ausgewählt ist aus T-Lymphozyten, NK-Zellen und Monozyten, bevorzugt T-Lymphozyten.

6. Antikörper nach Anspruch 3, wobei die weitere Bindungseinheit spezifisch an ein 10 bis 50 Aminosäuren langes Peptid, bevorzugt ein Peptid mit einer Aminosäuresequenz die einer 10 bis 50 Aminosäuren langen Teilsequenz des humanen La-Proteins entspricht, besonders bevorzugt ein Peptid mit einer der Aminosäuresequenzen gemäß SEQ ID No. 75 oder SEQ ID No. 76, bindet.

7. Antikörper nach einem der Ansprüche 3 bis 6 in Form eines Diabody, Triabody oder Tetrabody.

8. Antikörper nach einem der Ansprüche 1 bis 7 zur Verwendung als Arzneimittel, insbesondere zur Behandlung von Tumorerkrankungen, bevorzugt Prostatakrebs, oder als Diagnostikum.

9. Nukleinsäure, dessen Nukleotidsequenz für einen rekombinanten Antikörper nach einem der Ansprüche 1 bis 7 kodiert.

10. Vektor enthaltend eine Nukleotidsequenz nach Anspruch 9.

11. Wirtszelle oder nicht-menschlicher Wirtsorganismus enthaltend eine Nukleotidsequenz nach Anspruch 9 oder einen Vektor nach Anspruch 10.

12. Pharmazeutische Zusammensetzung enthaltend einen rekombinanten Antikörper nach einem der Ansprüche 1 bis 7 in Assoziation mit einem pharmazeutisch annehmbaren Verdünnungsmittel oder Träger, bevorzugt in einer für die intravenöse Verabreichung geeigneten Form.

13. Pharmazeutische Zusammensetzung nach Anspruch 12, enthaltend mindestens zwei unterschiedliche rekombinante Antikörper, davon mindestens einen Antikörper nach einem der Ansprüche 1 bis 7 und mindestens einen weiteren rekombinanten Antikörper, der mit dem Antikörper nach einem der Ansprüche 1 bis 7 eine spezifische Bindung eingeht.

14. Diagnostische Zusammensetzung enthaltend einen rekombinanten Antikörper nach einem der Ansprüche 1 bis 7.

## Claims

1. A prostate-specific stem cell antigen binding antibody, wherein

- the complementarity determining regions (CDR) of the variable region of the light chain comprises the following amino acid sequences: CDR1 SEQ ID No. 1, CDR2 SEQ ID No. 2, CDR3 SEQ ID No. 3, and
- the CDR of the variable region of the heavy chain comprises the following amino acid sequences: CDR1 SEQ ID No. 4, CDR2 SEQ ID No. 5, CDR3 SEQ ID No. 6.

2. Antibody according to claim 1 whose variable regions comprise a humanized amino acid sequence.

3. Antibody according to claim 1 or 2, comprising at least two different binding units, wherein

- at least one of the binding units binds to prostate-specific stem cell antigen and comprises the complementarity determining regions defined in claim 1, and
- at least one other one of the binding units binds specifically to an antigen other than prostate-specific stem cell antigen.

**4.** Antibody according to claim 3, wherein the other binding unit binds specifically to a surface structure on an effector cell, wherein the binding unit is preferably selected from a binding unit of an antibody or ligand binding to a surface structure of an effector cell.

**5.** Antibody according to claim 4, wherein the effector cell is selected from T lymphocytes, NK cells, and monocytes, preferably T lymphocytes.

**6.** Antibody according to claim 3, wherein the other binding unit specifically binds to a peptide of a length of 10 to 50 amino acids, preferably a peptide with an amino acid sequence that corresponds to a partial sequence of the human La protein with a length of 10 to 50 amino acids, particularly preferred a peptide with one of the amino acid sequences according to SEQ ID No. 75 or SEQ ID No. 76.

**7.** Antibody according to one of the claims 3 to 6 in the form of a diabody, triabody or tetrabody.

**8.** Antibodies according to one of the claims 1 to 7 for use as a medicament, in particular for the treatment of tumor diseases, preferably prostate cancer, or as a diagnostic agent.

**9.** Nucleic acid whose nucleotide sequence encodes for a recombinant antibody according to one of the claims 1 to 7.

**10.** Vector containing a nucleotide sequence according to claim 9.

**11.** Host cell or non-human host organism containing a nucleotide sequence according to claim 9 or a vector according to claim 10.

**12.** Pharmaceutical composition containing a recombinant antibody according to one of the claims 1 to 7 in association with a pharmaceutically acceptable thinner or carrier, preferably in a form suitable for intravenous administration.

**13.** Pharmaceutical composition according to claim 12, containing at least two different recombinant antibodies, at least one of them being an antibody according to one of the claims 1 to 7 and at least one other recombinant antibody which forms a specific binding with the antibody according to one of the claims 1 to 7.

**14.** Diagnostic composition containing a recombinant antibody according to one of the claims 1 to 7.

## Revendications

**1.** Anticorps se liant à un antigène de cellule-souche spécifique de la prostate, dans lequel

- les régions de détermination de la complémentarité (CDR) de la région variable de la chaîne légère comprennent les séquences d'acides aminés suivantes : CDR1 SEQ ID N° I, CDR2 SEQ ID N° 2, CDR3 SEQ ID N° 3 et
- les CDR de la région variable de la chaîne lourde comprennent les séquences d'acides aminés suivantes : CDR1 SEQ ID N° 4, CDR2 SEQ ID N° 5, CDR3 SEQ ID N° 6.

**2.** Anticorps selon la revendication 1, dont les régions variables présentent une séquence d'acides aminés humanisée.

**3.** Anticorps selon la revendication 1 ou 2, comprenant au moins deux éléments de liaison différents, dans lequel

- au moins un des éléments de liaison se lie à l'antigène de cellule-souche spécifique de la prostate et comprend les régions de détermination de la complémentarité définies dans la revendication 1 et
- au moins un élément de liaison supplémentaire se lie spécifiquement à un antigène autre que l'antigène de cellule-souche spécifique de la prostate.

**4.** Anticorps selon la revendication 3, dans lequel l'élément de liaison supplémentaire se lie spécifiquement à une structure de surface sur une cellule effectrice, dans lequel l'élément de liaison est de préférence sélectionné parmi un élément de liaison d'un anticorps ou de ligands se liant à une structure de surface d'une cellule effectrice.

5. Anticorps selon la revendication 4, dans lequel la cellule effectrice est sélectionnée parmi les lymphocytes T, les cellules NK et les monocytes, de préférence les lymphocytes T.

6. Anticorps selon la revendication 3, dans lequel l'élément de liaison supplémentaire se lie spécifiquement à un peptide d'une longueur de 10 à 50 acides aminés, de préférence un peptide doté d'une séquence d'acides aminés correspondant à une séquence partielle d'une longueur de 10 à 50 acides aminés de la protéine La humaine, plus préférablement un peptide doté d'une des séquences d'acides aminés selon SEQ ID N° 75 ou SEQ ID N° 76.

7. Anticorps selon l'une des revendications 3 à 6, sous forme d'un diabody, d'un triabody ou d'un tetrabody.

8. Anticorps selon l'une des revendications 1 à 7, pour une utilisation en tant que médicament, en particulier pour le traitement des tumeurs, de préférence le cancer de la prostate, ou en tant que diagnostic.

9. Acide nucléique, dont la séquence de nucléotides code pour un anticorps recombinant selon l'une des revendications 1 à 7.

10. Vecteur contenant une séquence de nucléotides selon la revendication 9.

11. Cellule hôte ou organisme hôte non humain contenant une séquence de nucléotides selon la revendication 9 ou un vecteur selon la revendication 10.

12. Composition pharmaceutique contenant un anticorps recombinant selon l'une des revendications 1 à 7 en association avec un diluant ou un support pharmaceutiquement acceptable, de préférence sous une forme adaptée à l'administration intraveineuse.

13. Composition pharmaceutique selon la revendication 12, contenant au moins deux anticorps recombinants différents, dont au moins un anticorps selon l'une des revendications 1 à 7 et au moins un anticorps recombinant supplémentaire, entrant dans une liaison spécifique avec l'anticorps selon l'une des revendications 1 à 7.

14. Composition diagnostique contenant un anticorps recombinant selon l'une des revendications 1 à 7.

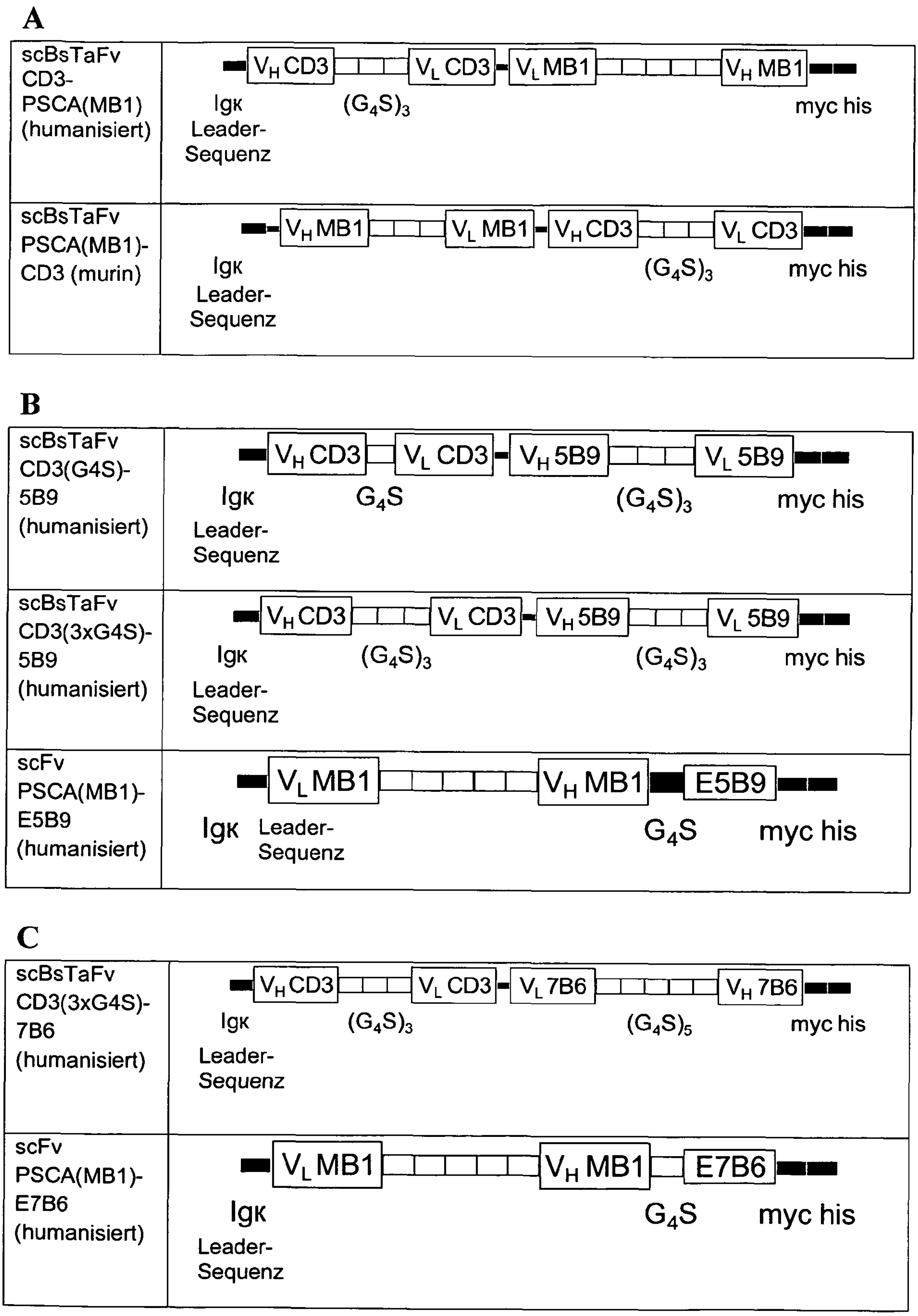
A
scBsTaFv CD3-PSCA(MB1) (humanisiert)
VH CD3
VL CD3
VL MB1
VH MB1
Igκ Leader-Sequenz
(G4S)3
myc his
scBsTaFv PSCA(MB1)-CD3 (murin)
VH MB1
VL MB1
VH CD3
VL CD3
Igκ Leader-Sequenz
(G4S)3
myc his
B
scBsTaFv CD3(G4S)-5B9 (humanisiert)
VH CD3
VL CD3
VH 5B9
VL 5B9
Igκ Leader-Sequenz
G4S
(G4S)3
myc his
scBsTaFv CD3(3xG4S)-5B9 (humanisiert)
VH CD3
VL CD3
VH 5B9
VL 5B9
Igκ Leader-Sequenz
(G4S)3
(G4S)3
myc his
scFv PSCA(MB1)-E5B9 (humanisiert)
VL MB1
VH MB1
E5B9
Igκ Leader-Sequenz
G4S
myc his
C
scBsTaFv CD3(3xG4S)-7B6 (humanisiert)
VH CD3
VL CD3
VL 7B6
VH 7B6
Igκ Leader-Sequenz
(G4S)3
(G4S)5
myc his
scFv PSCA(MB1)-E7B6 (humanisiert)
VL MB1
VH MB1
E7B6
Igκ Leader-Sequenz
G4S
myc his

**Fig. 1**

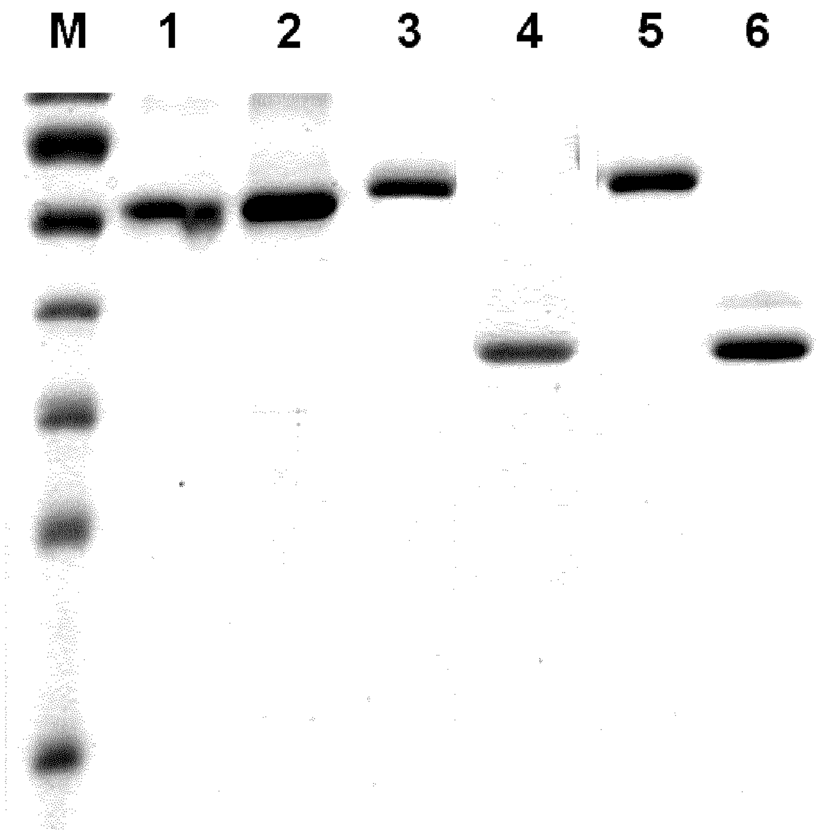


Fig. 2

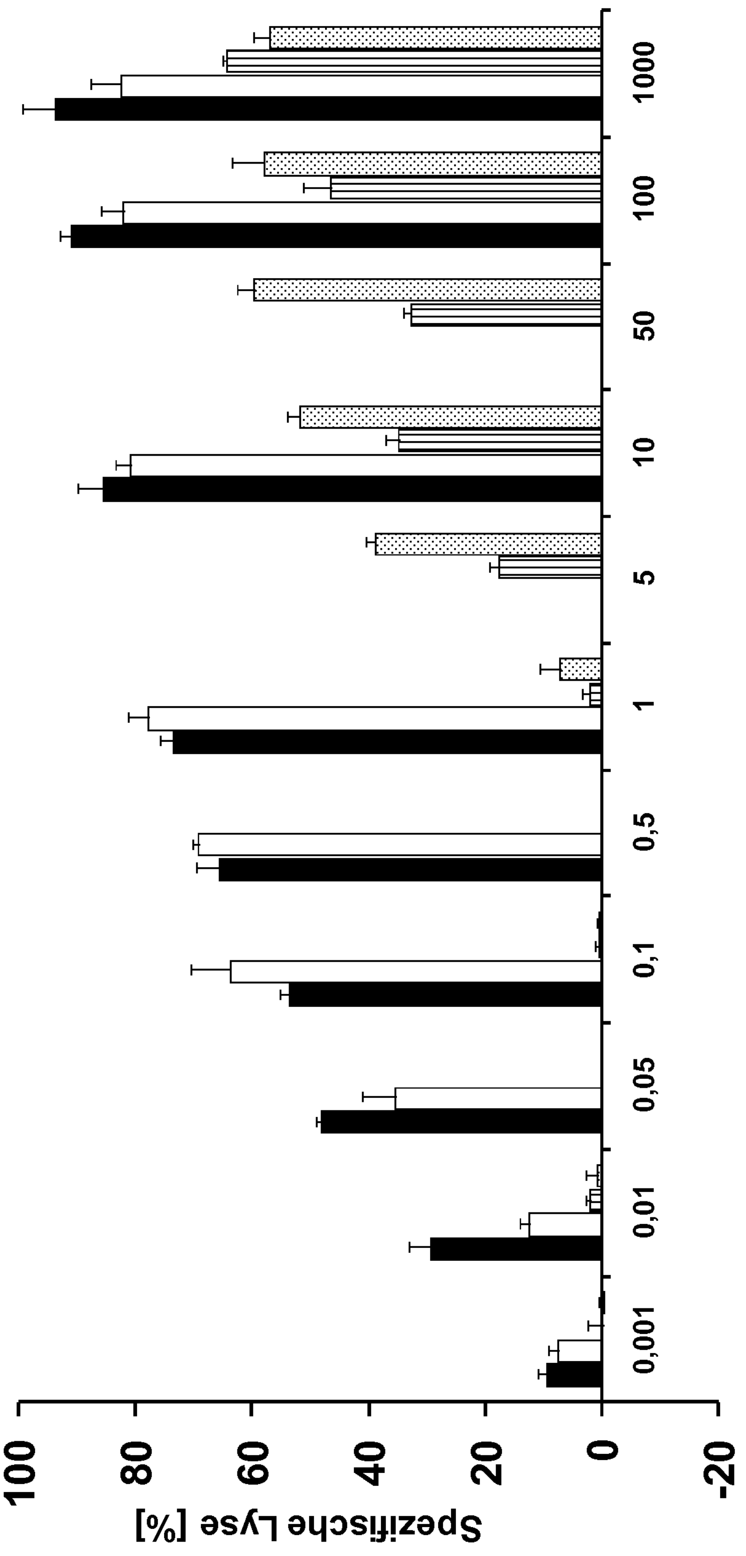
Spezifische Lyse [%]
100
80
60
40
20
0
-20
0,001
0,01
0,05
0,1
0,5
1
5
10
50
100
1000

Fig. 3

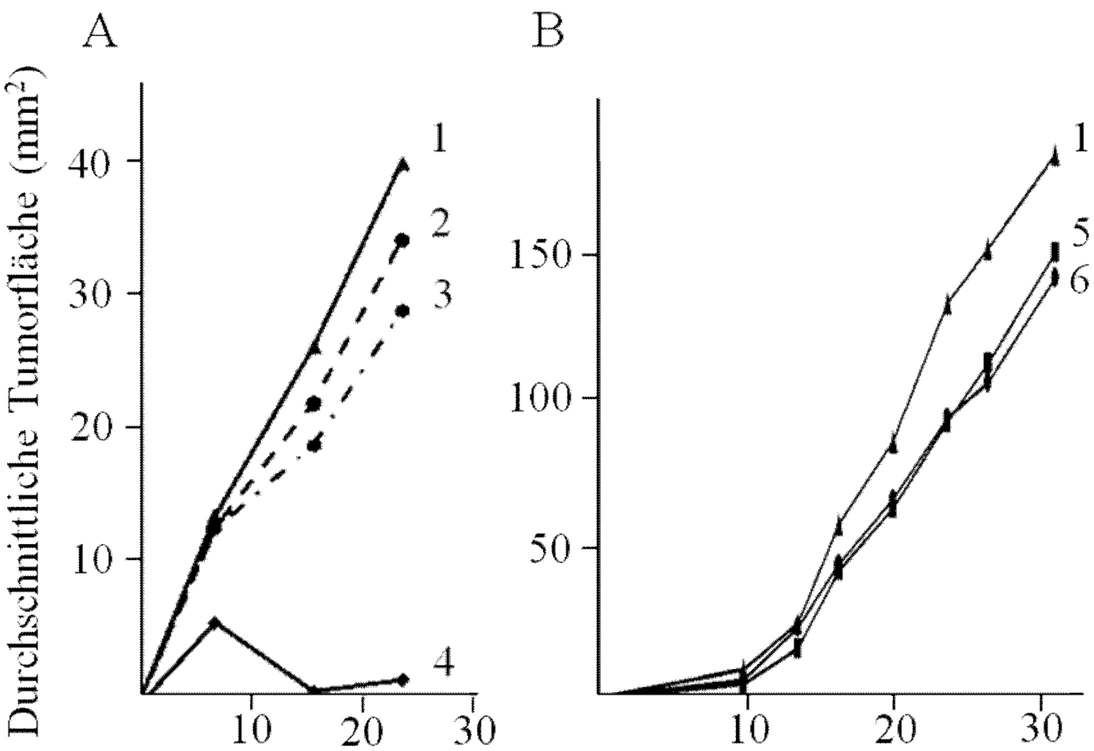
A
B
Durchschnittliche Tumorfläche (mm2)
40
30
20
10
10
20
30
1
2
3
4
150
100
50
10
20
30
1
5
6

**Fig. 4**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009032949 A2
- DE 102011118022


### In der Beschreibung aufgeführte Nicht-Patentliteratur


- **FELDMANN A ; STAMOVA S ; BIPPES CC ; BARTSCH H ; WEHNER R ; SCHMITZ M ; TEMME A ; CARTELLIERI M ; BACHMANN M.** Retargeting of T cells to prostate stem cell antigen expressing tumor cells: comparison of different antibody formats. *Prostate,* 15. Juni 2011, vol. 71 (9), 998-1011
- **GU Z ; YAMASHIRO J ; KONO E ; REITER RE.** Anti-prostate stem cell antigen monoclonal antibody 1G8 induces cell death in vitro and inhibits tumor growth in vivo via a Fc-independent mechanism. *Cancer Res.,* 15. Oktober 2005, vol. 65 (20), 9495-500
- **MORGENROTH A ; CARTELLIERI M ; SCHMITZ M ; GÜNES S ; WEIGLE B ; BACHMANN M ; ABKEN H ; RIEBER EP ; TEMME A.** Targeting of tumor cells expressing the prostate stem cell antigen (PSCA) using genetically engineered T-cells. *Prostate,* 01. Juli 2007, vol. 67 (10), 1121-31
- **REITER, R.E. ; GU, Z. ; WATABE, T. ; THOMAS, G. ; SZIGETI, K. ; DAVIS, E. ; WAHL, M. ; NISITANI, S. ; YAMASHIRO, J. ; LE BEAU, M.M.** Prostate stem cell antigen: A cell surface marker overexpressed in prostate cancer. *Proc Natl Acad Sci USA.,* 1998, vol. 95 (4), 1735-40
- **ROEHL, K.A. ; M. HAN ; C. G. RAMOS ; J. A. V. ANTENOR ; W. J. CATALONA.** Cancer progression and survival rates following anatomical radical retropubic prostatectomy in 3,478 consecutive patients: long-term results. *J Urol,* September 2004, vol. 172 (3), 910-4
- **THOMAS-KASKEL, A.-K. ; R. ZEISER ; R. JOCHIM ; C. ROBBEL ; W. SCHULTZE-SEEMANN ; C. F. WALLER ; H. VEELKEN.** Vaccination of advanced prostate cancer patients with PSCA and PSA peptide-loaded dendritic cells induces DTH responses that correlate with superior overall survival. *Int J Cancer,* November 2006, vol. 119 (10), 2428-34